(19) Europäisches Patentamt / European Patent Office / Office européen des brevets

(11) **EP 3 737 429 B1**

(12) **EUROPÄISCHE PATENTSCHRIFT**

(45) Veröffentlichungstag und Bekanntmachung des Hinweises auf die Patenterteilung:
**20.03.2024  Patentblatt 2024/12**

(21) Anmeldenummer: **19700111.8**

(22) Anmeldetag: **08.01.2019**

(51) Internationale Patentklassifikation (IPC):
**A61L 15/26** (2006.01)    **C08G 18/48** (2006.01)
**C08G 18/72** (2006.01)    **C08G 18/73** (2006.01)
**C08G 18/79** (2006.01)    **C08G 18/10** (2006.01)
**A61L 15/42** (2006.01)    **A61F 13/00** (2024.01)
**C08G 101/00** (2006.01)

(52) Gemeinsame Patentklassifikation (CPC):
(C-Sets verfügbar)
**C08G 18/10; A61L 15/26; A61L 15/425;**
**C08G 18/485; C08G 18/722; C08G 18/73;**
**C08G 18/792;** A61F 2013/0074; C08G 2110/0008;
C08G 2110/0058; C08G 2110/0066    (Forts.)

(86) Internationale Anmeldenummer:
**PCT/EP2019/050278**

(87) Internationale Veröffentlichungsnummer:
**WO 2019/137882 (18.07.2019 Gazette 2019/29)**

(54) **VERFAHREN ZUR HERSTELLUNG ELASTISCHER UND REISSFESTER POLYURETHANSCHÄUME SOWIE DEREN ANWENDUNGEN**

METHOD FOR THE MANUFACTURE OF ELASTIC AND TEAR-RESISTANT POLYURETHANE FOAMS AND THEIR APPLICATIONS

PROCÉDÉ DE PRODUCTION DE MOUSSES DE POLYURÉTHANE ÉLASTIQUES ET RÉSISTANTES À LA DÉCHIRURE AINSI QUE LEURS APPLICATIONS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **12.01.2018  EP 18151353**

(43) Veröffentlichungstag der Anmeldung:
**18.11.2020  Patentblatt 2020/47**

(73) Patentinhaber: **Covestro Deutschland AG**
**51373 Leverkusen (DE)**

(72) Erfinder:
• **SUETTERLIN, Jan**
  **51061 Köln (DE)**
• **WEISER, Marc-Stephan**
  **51515 Kürten-Dürscheid (DE)**
• **PLUG, Sascha**
  **51375 Leverkusen (DE)**
• **DÖRR, Sebastian**
  **40593 Düsseldorf (DE)**
• **STOYE, Claudine**
  **51067 Köln (DE)**

(74) Vertreter: **Levpat**
**c/o Covestro AG**
**Gebäude 4825**
**51365 Leverkusen (DE)**

(56) Entgegenhaltungen:
**US-A1- 2013 261 200    US-A1- 2014 107 243**

(52)  Gemeinsame Patentklassifikation (CPC): (Forts.)

C-Sets
**A61L 15/26, C08L 75/04;**
**C08G 18/10, C08G 18/302**

C-Sets
**A61L 15/26, C08L 75/04;**
**C08G 18/10, C08G 18/302**

**Beschreibung**

Die Erfindung betrifft ein Verfahren zur Herstellung von Polyurethanschäumen, bei dem Zusammensetzungen umfassend isocyanatfunktionelle Präpolymere A), Wasser B), optional heterocyclische, 4-Ring- oder 6-Ring-Oligomere von niedermolekularen Diisocyanaten mit einer Molmasse von 140 bis 278 g/mol C), optional Katalysatoren D), optional Salze schwacher Säuren deren korrespondierende freie Säuren in Wasser bei 25 °C einen pKS-Wert von ≥ 3,0 und ≤ 14,0 aufweisen, E), optional Tenside F), optional ein- oder mehrwertige Alkohole oder Polyole G), optional hydrophile Polyisocyanate H), wobei die isocyanathaltigen Komponenten, insbesondere die Komponenten A), C) und H), insgesamt einen Isocyanatgehalt in einem Bereich von 2 bis 8 Gew.-% und einen Gehalt an Urethangruppen von 1,0 bis 3,5 mol/kg, jeweils bezogen auf die Gesamtmenge der isocyanathaltigen Komponenten A) C) und H), aufweisen; sowie die so hergestellten Schäume und deren Verwendungen.

Im Stand der Technik sind Verfahren bekannt wie durch Mischen von polyisocyanathaltigen Ausgangsstoffen zusammen mit Wasser Schäume hergestellt werden können. Insbesondere werden in den Patentanmeldungen EP2143744, EP2470580, EP2585505 und EP2632501 solche Verfahren beschrieben unter Verwendung von Präpolymerformulierungen mit aliphatischen Isocyanaten, die einen Isocyanatgehalt in einem Bereich von 2 bis 8 Gew.-% aufweisen. Es wird in allen beschriebenen Beispielen ausschließlich ein Urethangehalt von kleiner 1,0 mol/kg verwendet. Das NCO/OH-Verhältnis während der Präpolymersynthese liegt bei den Beispielen bei einem Wert von 15. In keinem der oben erwähnten Dokumente werden mechanische Eigenschaften der hergestellten Schäume beschrieben.

Daneben sind Schäume auf Basis von aromatischen Polyisocyanaten bekannt. So werden in den Patentanmeldungen US3903232, US4137200, US5849850 und US2006142529 Präpolymerformulierungen verwendet, die entweder Isocyanatgehalte > 8 Gew.-% oder Urethangehalte < 1,0 mol/kg aufweisen.

Ein bisher nicht erreichtes Ziel bei der Herstellung solcher Schäume, insbesondere bei deren Einsatz in medizinischen Anwendungen ist es, verschiedene Eigenschaften, wie die Flexibilität des Schaums, die Absorptionsmenge von Flüssigkeiten, die Reißfestigkeit sowie ein nicht zu hohes Raumgewicht des Schaums gemeinsam zu optimieren. Nur so wäre es jedoch möglich einen möglichst reißfesten, aber dennoch flexiblen Schaum bereitzustellen, der dabei eine hohe Absorption für Flüssigkeiten bei möglichst geringem Gewicht aufweist. Insbesondere, um einen solchen Schaum beispielsweise für Wundschäume einsetzen zu können, wäre die Optimierung aller genannten Eigenschaften wünschenswert. Bisher hat sich jedoch gezeigt, dass ein Schaum mit hoher Absorptionsfähigkeit für wässrige Medien (im Folgenden auch Flüssigkeitsabsorption genannt) eine eher niedrige Flexibilität oder Bruchspannung aufweist. Daher besteht ein Bedarf, Schäume mit einer Kombination optimierter Eigenschaften bereitzustellen.

Eine Aufgabe der vorliegenden Erfindung war es, mindestens einen Nachteil des Standes der Technik mindestens zu einem Teil zu überwinden.

Eine weitere Aufgabe der vorliegenden Erfindung war es, einen Schaum bzw. ein Verfahren zur Herstellung dieses Schaums bereitzustellen, sodass der Schaum bei guter Absorption von Flüssigkeiten weiterhin eine hohe Flexibilität, also eine leichte Verformbarkeit, sowie eine hohe Bruchspannung besitzt, um möglichst strapazierfähig und dennoch flexibel zu sein. Insbesondere sollte der Schaum einen F20-Wert von ≤ 50 kPa, oder bevorzugt ≤ 30 kPa aufweisen. Der F20-Wert entspricht der Spannung bei 20% Dehnung im Zug-Dehnungsversuch nach DIN EN ISO 527-2, wie unter Methoden weiter ausgeführt.

Weiterhin war es eine Aufgabe der vorliegenden Erfindung, einen Schaum bzw. ein Verfahren zur Herstellung dieses Schaums bereitzustellen, sodass der Schaum einen Quotienten von Bruchspannung zu F20-Wert von mindestens 3,5, bevorzugt von mindestens 4,0, bevorzugt von mindestens 5,0 oder bevorzugt von mindestens 7,5 und dabei weiterhin eine hohe Flexibilität aufweist. Insbesondere sollte der Schaum dabei eine Dichte von 300 g/L, oder bevorzugt von 200 g/L nicht überschreiten.

Es war weiterhin eine Aufgabe der vorliegenden Erfindung, einen Schaum bzw. ein Verfahren zur Herstellung dieses Schaums bereitzustellen, der es ermöglicht einen aufrollbaren Schaum in Form von Schaumbahnen herzustellen, ohne dass die Schaumbahnen unter den beim Aufrollen auftretenden Zugkräften reißt. Bevorzugt sollen die Schäume eine Bruchspannung von ≥ 50 kPa, oder bevorzugt ≥ 100 kPa aufweisen.

Weiterhin war es eine Aufgabe einen Schaum sowie ein Verfahren zur Herstellung des Schaums bereitzustellen, bei dem der Schaum eine hohe Flüssigkeitsabsorption aufweist, bevorzugt in einem Bereich von 530 bis 4000 %, oder bevorzugt von 800 bis 3500 %, oder bevorzugt von 1000 bis 3000 %, bezogen auf den ursprünglichen Flüssigkeitsgehalt. Dabei soll der Schaum eine angenehme Haptik aufweisen und sich leicht an gekrümmte Oberflächen anpassen können.

Überraschenderweise konnte gefunden werden, dass die Merkmalskombination des Gegenstandes des Anspruch 1 mindestens eine der Aufgaben lösen konnte.

Ein erster Gegenstand der Erfindung betrifft ein Verfahren zur Herstellung von Polyurethanschäumen, bei dem Zusammensetzungen umfassend

    A) Isocyanatfunktionelle Präpolymere erhältlich durch die Umsetzung von

A1) niedermolekularen Diisocyanaten einer Molmasse von 140 bis 278 g/mol, mit

A2) Polyalkylenoxiden mit einer OH-Funktionalität von zwei oder mehr, bevorzugt in einem Bereich von 2 bis 6, bevorzugt in einem Bereich von 2 bis 5, oder bevorzugt 2 bis 4,

A3) optional weitere isocyanatreaktive Komponenten, die nicht unter A2) fallen;

B) Wasser in einer Menge von mindestens 2 Gew.-%, bevorzugt von mindestens 5 Gew.-%, oder bevorzugt von mindestens 10 Gew.-%, oder bevorzugt in einem Bereich von 2 bis 40 Gew.-%, oder bevorzugt in einem Bereich von 5 bis 30 Gew.-%, oder bevorzugt in einem Bereich von 10 bis 25 Gew.-%, bezogen auf das Gesamtgewicht der Zusammensetzung;

C) optional heterocyclische, 4-Ring- oder 6-Ring-Oligomere von niedermolekularen Diisocyanaten mit einer Molmasse von 140 bis 278 g/mol;

D) optional Katalysatoren;

E) optional Salze schwacher Säuren deren korrespondierende freie Säuren in Wasser bei 25 °C einen pKS-Wert von $\geq$ 3,0 und $\leq$ 14,0 aufweisen;

F) optional Tenside; und

G) optional ein- oder mehrwertige Alkohole oder Polyole;

H) optional hydrophile Polyisocyanate erhältlich durch Umsetzung von

H1) niedermolekularen Diisocyanaten einer Molmasse von 140 bis 278 g/mol und / oder daraus herstellbaren Polyisocyanaten mit einer Isocyanatfunktionalität von 2 bis 6 mit

H2) monofunktionellen Polyalkylenoxiden einer OH-Zahl von 10 bis 250, und einem Ethylenoxidanteil von 50 bis 100 mol% bezogen auf die Gesamtmenge der enthaltenen Oxyalkylengruppen.

bereitgestellt, aufgeschäumt und ausgehärtet werden,

wobei die isocyanathaltigen Komponenten, insbesondere die Komponenten A, C und H, insgesamt einen Isocyanatgehalt in einem Bereich von 2 bis 8 Gew.-% und einen Gehalt an Urethangruppen von 1,0 bis 3,5 mol/kg, jeweils bezogen auf die Gesamtmenge der isocyanathaltigen Komponenten, aufweisen. Bevorzugt weisen die isocyanathaltigen Komponenten, insbesondere die Komponenten A, C und H, insgesamt einen Isocyanatgehalt in einem Bereich von 3 bis 7, oder bevorzugt in einem Bereich von 4 bis 6,5 Gew.-% und bevorzugt einen Gehalt an Urethangruppen in einem Bereich von 1,5 bis 3,0 mol/kg, oder bevorzugt in einem Bereich von 1,7 bis 2,8 mol/kg, jeweils bezogen auf die Gesamtmenge der isocyanathaltigen Komponenten, auf.

Bevorzugt weisen die eingesetzten Präpolymere A) einen Restmonomergehalt von unter 0,5 Gew.-% bezogen auf das Präpolymer auf. Dieser Gehalt kann durch entsprechend gewählte Einsatzmengen der Diisocyanate A1) und der Polyalkylenoxide A2) erreicht werden. Bevorzugt ist jedoch der Einsatz des Diisocyanats A1) im Überschuss und mit anschließender, bevorzugt destillativer, Abtrennung nicht umgesetzter Monomere.

Bei der Herstellung der isocyanatfunktionellen Präpolymere A) wird das Verhältnis der Polyalkylenoxide A2) zu den niedermolekularen, aliphatischen Diisocyanaten A1) typischerweise so eingestellt, dass auf 1 Mol OH-Gruppen des Polyalkylenoxide A2) 1,1 bis 20 Mol, bevorzugt mit 1,3 bis 5 Mol und besonders bevorzugt 1,5 bis 3,5 Mol NCO-Gruppen des niedermolekularen, aliphatischen Diisocyanats A1) kommen.

Die Umsetzung kann in Gegenwart von Urethanisierungskatalysatoren wie Zinnverbindungen, Zinkverbindungen, Aminen, Guanidinen oder Amidinen, oder in Gegenwart von Allophanatisierungskatalysatoren wie Zinkverbindungen erfolgen.

Die Umsetzung erfolgt typischerweise bei 25 bis 140 °C, bevorzugt 60 bis 100 °C.

Wurde mit überschüssigem Isocyanat gearbeitet, so erfolgt anschließend die Entfernung des Überschusses an niedermolekularem, aliphatischen Diisocyanat bevorzugt durch Dünnschichtdestillation.

Bevorzugt werden vor, während und nach der Reaktion oder der destillativen Abtrennung des Diisocyanatüberschusses saure oder alkylierende Stabilisatoren, wie Benzoylchlorid, Isophthaloylchlorid, Methyltosylat, Chlorpropionsäure, HCl, Dibutylphosphat oder Antioxidantien wie Di-tert-butylkresol oder Tocopherol zugesetzt.

Der NCO-Gehalt der isocyanatfunktionellen Präpolymere A) beträgt bevorzugt 1,5 bis 8 Gew.-%, besonders bevorzugt 2 bis 7,5 Gew.-% und ganz besonders bevorzugt 3 bis 7 Gew.-%.

Beispiele für niedermolekulare, aliphatische Diisocyanate der Komponente A1) sind Hexamethylendiisocyanat (HDI), Isophorondiisocyanat (IPDI), Butylendiisocyanat (BDI), Pentamethylendiisocyanat (PDI), Bisisocyanatocyclohexylmethan (HMDI), 2,2,4-Trimethylhexamethylendiisocyanat, Bisisocyanatomethylcyclohexan, Bisisocyanatomethyltricyclodecan, Xylendiisocyanat, Tetramethylxylylendiisocyanat, Norbornandiisocyanat, Cyclohexandiisocyanat oder Diisocyanatododecan, wobei Hexamethylendiisocyanat (HDI), Isophorondiisocyanat (IPDI), Butylendiisocyanat (BDI), Pentamethylendiisocyanat (PDI), und Bis-(isocyanatocyclohexyl)methan (HMDI) bevorzugt sind. Besonders bevorzugt sind PDI, HDI, IPDI, ganz besonders bevorzugt Hexamethylendiisocyanat und Pentamethylendiisocyanat.

Werden aromatische Diisocyanate als Komponente A1 eingesetzt so handelt es sich bevorzugt um Toluol-2,4-diisocyanat (2,4-TDI), Toluol-2,6-diisocyanat (2,6-TDI) oder 2,2'-Methylendiphenyldiisocyanat (2,2'-MDI), 2,4'-Methylendiphenyldiisocyanat (2,4'-MDI), 4,4'-Methylendiphenyldiisocyanat (4,4'-MDI), oder Mischungen aus mindestens zwei hiervon.

Polyalkylenoxide der Komponente A2) können alle Polyalkylenoxide sein, die der Fachmann hierfür verwenden würde. Beispiele hierfür sind ausgewählt aus der Gruppe bestehend aus Polyethylenoxid, Polypropylenoxid, Polytetrahydrofuran oder einer Mischung aus mindestens zwei hiervon. Polyalkylenoxide der Komponente A2) sind bevorzugt Copolymere aus Ethylenoxid und Propylenoxid mit einem Ethylenoxidgehalt, bezogen auf die Gesamtmenge der enthaltenen Oxyalkylengruppen, von 50 bis 100 mol%, bevorzugt von 60 bis 90 mol%, gestartet auf Polyolen oder Aminen. Bevorzugte Starter dieser Art sind ausgewählt aus der Gruppe bestehend aus Ethylenglycol, 1,3-Propandiol, 1,4-Butandiol, Glycerin, Trimethylolpropan (TMP), Sorbit, Pentaerythrit, Triethanolamin, Ammoniak und Ethylendiamin oder eine Mischung aus mindestens zwei hiervon.

Die Polyalkylenoxide der Komponente A2) besitzen typischerweise zahlenmittlere Molekulargewichte von 250 bis 10000 g/mol, bevorzugt von 300 bis 2800 g/mol, oder bevorzugt 350 bis 1500 g/mol.

Ferner besitzen die Polyalkylenoxide der Komponente A2) OH-Funktionalitäten von 2 bis 6, bevorzugt von 2 bis 5, besonders bevorzugt von 2 bis 4.

Bevorzugt werden für die Komponente A3) grundsätzlich alle dem Fachmann an sich bekannten ein- und mehrwertigen Alkohole oder Amine sowie Mischungen hieraus eingesetzt. Dies sind ein- oder mehrwertige Alkohole oder Polyole, wie Ethanol, Propanol, Butanol, Decanol, Tridecanol, Hexadecanol, Ethylenglykol, Neopentylglykol, Butandiol, Hexandiol, Decandiol, Trimethylolpropan, Glycerin, Pentaerythrit, monofunktionelle Polyetheralkohole und Polyesteralkohole, Polyetherdiole und Polyesterdiole oder Mischungen von mindestens zwei hiervon. Beispiele für ein- oder mehrwertige Amine seien Butylamin Ethylendiamin oder aminterminierte Polyalkylenglycole (z.B. Jeffamine®) genannt.

Bei der Herstellung des Präpolymer A) können Katalysatoren eingesetzt, wie sie beispielsweise für Komponente D) beschrieben werden.

Das als Komponente B) einzusetzende Wasser kann als solches, als Kristallwasser eines Salzes, als Lösung in einem dipolar-aprotischen Lösungsmittel oder auch als Emulsion eingesetzt werden. Bevorzugt wird das Wasser als solches oder in einem dipolar-aprotischen Lösungsmittel eingesetzt. Ganz besonders bevorzugt wird das Wasser als solches eingesetzt.

Gegebenenfalls einzusetzende Verbindungen der Komponente C) sind heterocyclische, 4-Ring- oder 6-Ring-Oligomere von niedermolekularen Diisocyanaten mit einer Molmasse von 140 bis 278 g/mol wie Isocyanurate, Iminooxadiazindione oder Uretdione der vorgenannten niedermolekularen Diisocyanate. Bevorzugt werden für Komponente C) aliphatische Diisocyanate verwendet. Bevorzugt sind heterocyclische 4-Ring-Oligomere wie Uretdione.

Bevorzugt weisen die 4-Ring- oder 6-Ring-Oligomere von niedermolekularen Diisocyanaten eine Funktionalität in einem Bereich von 2 bis 6, oder bevorzugt in einem Bereich von 2,1 bis 5,5, oder bevorzugt in einem Bereich von 2,5 bis 5 auf.

Beispiele für niedermolekulare, aliphatische Diisocyanate der Komponente C) sind Hexamethylendiisocyanat (HDI), Isophorondiisocyanat (IPDI), Butylendiisocyanat (BDI), Pentamethylendiisocyanat (PDI), Bisisocyanatocyclohexylmethan (HMDI), 2,2,4-Trimethylhexamethylendiisocyanat, Bisisocyanatomethylcyclohexan, Bisisocyanatomethyltricyclodecan, Xylendiisocyanat, Tetramethylxylylendiisocyanat, Norbornandiisocyanat, Cyclohexandiisocyanat oder Diisocyanatododecan, wobei Hexamethylendiisocyanat (HDI), Isophorondiisocyanat (IPDI), Butylendiisocyanat (BDI), Pentamethylendiisocyanat (PDI), und Bis(isocyanatocyclohexyl)methan (HMDI) bevorzugt sind. Besonders bevorzugt sind PDI, HDI, IPDI, ganz besonders bevorzugt Hexamethylendiisocyanat und Pentamethylendiisocyanat.

Werden aromatische Diisocyanate als Komponente C) eingesetzt so handelt es sich bevorzugt um Toluol-2,4-diisocyanat (2,4-TDI), Toluol-2,6-diisocyanat (2,6-TDI) oder 2,2'-Methylendiphenyldiisocyanat (2,2'-MDI), 2,4'-Methylendiphenyldiisocyanat (2,4'-MDI), 4,4'-Methylendiphenyldiisocyanat (4,4'-MDI), oder Mischungen aus mindestens zwei hiervon.

Der durch den Einsatz der Komponente C) erhöhte Gehalt an Isocyanatgruppen sorgt für ein besseres Aufschäumen, da bei der Isocyanat-Wasser-Reaktion mehr $CO_2$ gebildet wird.

Zur Beschleunigung der Urethanbildung können in Komponente D) Katalysatoren eingesetzt werden. Dabei handelt es sich typischerweise um die dem Fachmann aus der Polyurethantechnologie bekannten Verbindungen. Bevorzugt sind hier Verbindungen der Gruppe bestehend aus katalytisch aktiven Metallsalzen, Aminen, Amidinen und Guanidinen. Beispielhaft zu nennen sind Zinndibutyldilaurat (DBTL), Zinnacetat, 1,8-Diazabicyclo[5.4.0]undecen-7 (DBU), 1,5-Diazabicyclo[4.3.0]nonen-5 (DBN), 1,4-Diazabicyclo[3.3.0]octen-4 (DBO), N-Ethylmorpholin (NEM), Triethylendiamin (DABCO), Pentamethylguanidin (PMG), Tetrametylguanidin (TMG), Cyclotetramethylguanidin (TMGC), n-Decyltetramethylguanidin (TMGD), n-Dodecyltetramethylguanidin (TMGDO), Dimethylaminoethyltetramethylguanidin (TMGN), 1,1,4,4,5,5-Hexamethylisobiguanidin (HMIB), Phenyltetramethylguanidin (TMGP) und Hexamethylenoctamethylbiguanidin (HOBG).

Bevorzugt ist der Einsatz von Aminen, Amidinen, Guanidinen oder deren Mischungen als Katalysatoren der Komponente D). Weiterhin bevorzugt ist auch die Verwendung von 1,8-Diazabi-cyclo[5.4.0]undecen-7 (DBU).

Bevorzugt wird in dem erfindungsgemäßen Verfahren ganz auf Katalysatoren verzichtet.

Gegebenenfalls können als Komponente E) Salze schwacher Säuren verwendet werden, deren korrespondierende freie Säuren in Wasser bei 25 °C einen pKS-Wert von $\geq 3,0$ und $\leq 14,0$ aufweisen. Beispiele geeigneter Salze schwacher Säuren sind Kaliumhydroxid, Natriumhydroxid, Kaliumcarbonat, Natriumcarbonat und Natriumhydrogencarbonat, Natriumacetat, Kaliumacetat, Natriumcitrat, Kaliumcitrat, Natriumbenzoat, Kaliumbenzoat, wobei auch beliebige Mischungen dieser Salze mit umfasst sind. Bevorzugt ist, wenn die Salze schwacher Säuren aus der Gruppe Natriumhydroxid, Natriumhydrogencarbonat und Natriumcarbonat ausgewählt sind. In diesem Fall resultiert eine besonders kurze Reaktionszeit.

Zur Verbesserung der Schaumbildung, Schaumstabilität oder der Eigenschaften des resultierenden Polyurethanschaums können Verbindungen der Komponente F) eingesetzt werden, wobei solche Additive grundsätzlich alle an sich bekannten anionischen, kationischen, amphoteren und nichtionischen Tenside sowie Mischungen hieraus sein können. Bevorzugt werden Alkylpolyglycoside, EO/PO-Blockcopolymere, Alkyl- oder Arylalkoxylate, Siloxanalkoxylate, Ester der Sulfobernsteinsäure und/oder Alkali- oder Erdalkalimetallalkanoate oder Mischungen von mindestens zwei hiervon eingesetzt. Besonders bevorzugt werden EO/PO-Blockcopolymere eingesetzt. Bevorzugt werden allein die EO/PO-Blockcopolymere als Komponente F) eingesetzt.

Zudem können zur Verbesserung der Schaumeigenschaften des resultierenden Polyurethanschaums Verbindungen der Komponente G) verwendet werden. Hierbei handelt es sich um grundsätzlich alle dem Fachmann an sich bekannten ein- und mehrwertigen Alkohole sowie Mischungen hieraus. Dies sind ein- oder mehrwertige Alkohole oder Polyole, wie Ethanol, Propanol, Butanol, Decanol, Tridecanol, Hexadecanol, Ethylenglykol, Neopentylglykol, Butandiol, Hexandiol, Decandiol, Trimethylolpropan, Glycerin, Pentaerythrit, monofunktionelle Polyetheralkohole und Polyesteralkohole, Polyetherdiole und Polyesterdiole oder Mischungen von mindestens zwei hiervon.

Bei der Herstellung der hydrophilen Polyisocyanate H) wird das Verhältnis der monofunktionellen Polyalkylenoxide H2) zu den niedermolekularen Diisocyanaten H1) typischerweise so eingestellt, dass auf 1 Mol OH-Gruppen der monofunktionellen Polyalkylenoxide 1,25 bis 20 Mol, bevorzugt mit 2 bis 15 Mol und besonders bevorzugt 5 bis 13 Mol NCO-Gruppen des niedermolekularen Diisocyanats H1) kommen. Anschließend erfolgt die Allophanatisierung bzw. Biurethisierung und/oder Isocyanuratbildung bzw. Uretdionbildung. Werden die Polyalkylenoxide H2) über Urethangruppen an die bevorzugt aliphatischen Diisocyanate H1) gebunden, findet bevorzugt im Anschluss eine Allophanatisierung statt. Weiterhin ist bevorzugt, dass Isocyanurat-Struktureinheiten gebildet werden.

Eine bevorzugte alternative Herstellung der hydrophilen Polyisocyanate H) erfolgt typischerweise durch Umsetzung von 1 Mol OH-Gruppen der monofunktionellen Polyalkylenoxidkomponente H2) mit 1,25 bis 20 Mol, bevorzugt mit 2 bis 15 Mol und besonders bevorzugt 5 bis 13 Mol NCO-Gruppen eines Polyisocyanates H1) mit einer Isocyanatfunktionalität von 2 bis 6, basierend auf aliphatischen oder aromatischen Diisocyanaten, bevorzugt aliphatischen, Diisocyanaten. Beispielhaft für derartige Polyisocyanate H1) sind Biuret-Strukturen, Isocyanurate bzw. Uretdione basierend auf bevorzugt aliphatischen Diisocyanaten. Dabei werden das Polyisocyanat H1) und das Polyalkylenoxid H2) bevorzugt über eine Urethangruppe bzw. eine Harnstoffgruppe miteinander verknüpft, wobei besonders die Verknüpfung über Urethangruppen bevorzugt ist.

Bevorzugt werden ausschließlich aliphatische Diisocyanate bei der Herstellung erfindungsgemäßer Polyurethanschäume eingesetzt. Insbesondere werden bevorzugt ausschließlich aliphatische Diisocyanate für die Komponenten A1), C) und H1) eingesetzt.

Die Umsetzung kann in Gegenwart von Urethanisierungskatalysatoren wie Zinnverbindungen, Zinkverbindungen, Aminen, Guanidinen oder Amidinen, oder in Gegenwart von Allophanatisierungskatalysatoren wie Zinkverbindungen erfolgen.

Die Umsetzung erfolgt typischerweise bei 25 bis 140 °C, bevorzugt bei 60 bis 100 °C.

Wurde mit überschüssigem niedermolekularen Diisocyanat gearbeitet, erfolgt anschließend die Entfernung des Überschusses an niedermolekularem, aliphatischen Diisocyanat bevorzugt durch Dünnschichtdestillation.

Vor, während und/oder nach der Reaktion oder der destillativen Abtrennung des Diisocyanatüberschusses können saure oder alkylierende Stabilisatoren, wie Benzoylchlorid, Isophthaloylchlorid, Methyltosylat, Chlorpropionsäure, HCl oder Antioxidantien, wie Di-*tert*-butylkresol oder Tocopherol zugesetzt werden.

Der NCO-Gehalt der hydrophilen Polyisocyanate H) beträgt bevorzugt 0,3 bis 23 Gew.-%, besonders bevorzugt 2 bis 21 Gew.-% und ganz besonders bevorzugt 3 bis 18 Gew.-%.

Beispiele für niedermolekulare, aliphatische Diisocyanate der Komponente H1) sind Hexamethylendiisocyanat (HDI), Isophorondiisocyanat (IPDI), Butylendiisocyanat (BDI), Pentamethylendiisocyanat (PDI), Bisisocyanatocyclohexylmethan (HMDI), 2,2,4-Trimethylhexamethylendiisocyanat, Bisisocyanatomethylcyclohexan, Bisisocyanatomethyltricyclodecan, Xylendiisocyanat, Tetramethylxylylendiisocyanat, Norbornandiisocyanat, Cyclohexandiisocyanat oder Diisocyanatododecan, wobei Hexamethylendiisocyanat (HDI), Isophorondiisocyanat (IPDI), Butylendiisocyanat (BDI), Pentamethylendiisocyanat (PDI), und Bis-(isocyanatocyclohexyl)methan (HMDI) bevorzugt sind. Besonders bevorzugt sind PDI, HDI, IPDI, ganz besonders bevorzugt Hexamethylendiisocyanat und Pentamethylendiisocyanat.

Werden aromatische Diisocyanate als Komponente H1) eingesetzt so handelt es sich bevorzugt um Toluol-2,4-diisocyanat (2,4-TDI), Toluol-2,6-diisocyanat (2,6-TDI) oder 2,2'-Methylendiphenyldiisocyanat (2,2'-MDI), 2,4'-Methylendiphenyldiisocyanat (2,4'-MDI), 4,4'-Methylendiphenyldiisocyanat (4,4'-MDI), oder Mischungen aus mindestens zwei hiervon.

Beispiele für höhermolekulare Polyisocyanate H2) sind Polyisocyanate mit einer Isocyanatfunktionalität von 2 bis 6 mit Isocyanurat-, Urethan-, Allophanat-, Biuret-, Iminooxadiazintrion-, Oxadiazintrion- und/oder Uretdiongruppen auf Basis der im vorstehenden Abschnitt genannten aliphatischen und/oder cycloaliphatischen Diisocyanate.

Bevorzugt werden als Komponente H2) höhermolekulare Verbindungen mit Biuret-, Iminooxadiazindion-, Isocyanurat- und/oder Uretdiongruppen auf Basis von Hexamethylendiisocyanat, Isophorondiisocyanat und/oder 4,4'-Diisocyanatodicyclohexylmethan eingesetzt. Weiter bevorzugt sind Isocyanurate. Ganz besonders bevorzugt sind Strukturen auf Basis von Hexamethylendiisocyanat.

Die monofunktionellen Polyalkylenoxiden H2) weisen eine OH-Zahl von 15 bis 250, bevorzugt von 28 bis 112, und einem Ethylenoxidanteil von 50 bis 100 mol%, bevorzugt von 60 bis 100 mol%, bezogen auf die Gesamtmenge der enthaltenen Oxyalkylengruppen auf.

Unter monofunktionellen Polyalkylenoxiden im Sinne der Erfindung sind Verbindungen zu verstehen, die nur eine isocyanatreaktive Gruppe, d.h. eine Gruppe, die mit einer NCO-Gruppe reagieren kann, aufweisen.

Die Herstellung von Polyalkylenoxiden H2) durch Alkoxylierung geeigneter Startermoleküle ist literaturbekannt (z.B. Ullmanns Encyclopädie der technischen Chemie, 4. Auflage, Band 19, Verlag Chemie, Weinheim S. 31-38). Geeignete Startermoleküle sind insbesondere gesättigte Monoalkohole wie Methanol, Ethanol, n-Propanol, Isopropanol, n-Butanol, Isobutanol, sec-Butanol, Diethylenglykolmonobutylether sowie aromatische Alkohole wie Phenol oder Monoamine wie Diethylamin. Bevorzugte Startermoleküle sind gesättigte Monoalkohole der vorstehend genannten Art. Besonders bevorzugt werden Diethylenglykolmonobutylether oder n-Butanol als Startermoleküle verwendet.

Die monofunktionellen Polyalkylenoxide H2) besitzen typischerweise zahlenmittlere Molekulargewichte von 220 bis 3700 g/mol, bevorzugt von 250 bis 2800 g/mol, oder bevorzugt von 300 bis 2000 g/mol.

Die monofunktionellen Polyalkylenoxide H2) besitzen bevorzugt eine OH-Gruppe als isocyanatreaktive Gruppe.

Typischerweise werden die Komponenten A) bis H) in folgenden Mengen eingesetzt:

100 Gewichtsteile isocyanatfunktioneller Präpolymere A)

0,1 bis 200 Gewichtsteile Wasser B)

0 bis 30 Gewichtsteile heterocyclischer Oligomere C)

0 bis 1 Gewichtsteile an Katalysatoren D)

0 bis 5 Gewichtsteile an Salzen schwacher Säuren deren korrespondierende freie Säuren in Wasser bei 25 °C einen pKS-Wert von $\geq 3,0$ und $\leq 14,0$ aufweisen E)

0 bis 10 Gewichtsteile Tenside F)

0 bis 20 Gewichtsteile Alkohole G)

0,5 bis 50 Gewichtsteilen hydrophile Polyisocyanatkomponente H)

Bevorzugt werden die Komponenten A) bis H) in folgenden Mengen eingesetzt:

100 Gewichtsteile isocyanatfunktioneller Präpolymere A)

0,1 bis 100 Gewichtsteile Wasser B)

1 bis 20 Gewichtsteile heterocyclischer Oligomere C)

0 bis 1 Gewichtsteile an Katalysatoren D)

0 bis 5 Gewichtsteile an Salzen schwacher Säuren deren korrespondierende freie Säuren in Wasser bei 25 °C einen pKS-Wert von ≥ 3,0 und ≤ 14,0 aufweisen E)

0 bis 5 Gewichtsteile Tenside F)

0 bis 10 Gewichtsteile Alkohole G)

1 bis 25 Gewichtsteilen hydrophile Polyisocyanate H)

Besonders bevorzugt werden die Komponenten A) bis H) in folgenden Mengen eingesetzt:

100 Gewichtsteile isocyanatfunktioneller Präpolymere A)

1 bis 60 Gewichtsteile Wasser B)

2 bis 15 Gewichtsteile heterocyclischer Oligomere C)

0 bis 0,5 Gewichtsteile an Katalysatoren D)

0 Gewichtsteile an Salzen schwacher Säuren deren korrespondierende freie Säuren in Wasser bei 25 °C einen pKS-Wert von ≥ 3,0 und ≤ 14,0 aufweisen E)

0 bis 3 Gewichtsteile Tenside F)

0 Gewichtsteile Alkohole G)

2 bis 15 Gewichtsteilen hydrophile Polyisocyanate H)

In einer bevorzugten Ausführungsform des erfindungsgemäßen Verfahrens weist die Komponente A) einen Gewichtsanteil an niedermolekularen Diisocyanaten mit einer Molmasse von 140 bis 278 g/mol von unter 1,0 Gew.-%, bevorzugt von unter 0,5 Gew.-%, bevorzugt von unter 0,3 Gew.-%, oder bevorzugt von unter 0,1 Gew.-%, bezogen auf das Präpolymer auf. Die Einstellung des Gewichtsanteils an niedermolekularen Diisocyanaten erfolgt bevorzugt über Destillation.

In einer bevorzugten Ausführungsform des erfindungsgemäßen Verfahrens werden aliphatische Isocyanate als Komponente A1) eingesetzt.

In einer bevorzugten Ausführungsform des erfindungsgemäßen Verfahrens werden mindestens folgende Schritte durchgeführt:

I) Herstellen des Präpolymers A) aus den Komponenten A1), A2) und gegebenenfalls A3), D),

II) Optionales Mischen der Komponenten A), C) und H) sowie anderer isocyanathaltiger Komponenten unter Erhalt einer Präpolymer-Mischung,

III) Optional Zugabe von A3) und gegebenenfalls D),

IV) Optionales Mischen der Komponente B) mit allen weiteren Komponenten, insbesondere D), E), F) und G) außer der Präpolymer-Mischung,

V) Mischen der unter I) bis III) erhaltenen Präpolymer-Mischung mit der Mischung aus IV).

Bevorzugt wird die Temperatur bei mindestens einem der Schritte I) bis IV) in einem Bereich von 2 bis 70°C, oder bevorzugt in einem Bereich von 10 bis 50 °C, oder bevorzugt von 20 bis 40 °C gewählt.

Bevorzugt wird die Mischung im Anschluss an Schritt IV) auf ein Substrat aufgegeben und aushärten gelassen. Das Aushärten wird bevorzugt bei einer Temperatur in einem Bereich von 20 bis 50 °C durchgeführt. Unter zu Hilfenahme von Konvektionsöfen oder Infrarottrocknern kann das Aushärten und eine gleichzeitige Trocknung auch bei höheren Temperaturbereichen, z.B. zwischen 50 und 200 °C durchgeführt werden.

Die Herstellung der erfindungsgemäßen Polyurethanschäume erfolgt bevorzugt durch Mischen der Komponenten A), hergestellt aus den Komponenten A1), A2) und gegebenenfalls A3), D), gegebenenfalls mit C) und/oder H) in beliebiger Reihenfolge, sowie anschließend mit einer Mischung aus B) und gegebenenfalls D), E), F), G), Aufschäumen der Mischung und Aushärtung, bevorzugt durch chemische Vernetzung. Bevorzugt werden die Komponenten A), C) und H) miteinander vorvermischt. Die gegebenenfalls einzusetzenden Salze E) und gegebenenfalls die Tenside F) werden bevorzugt in Form ihrer wässrigen Lösungen dem Reaktionsgemisch zugesetzt.

Das Aufschäumen kann dabei grundsätzlich durch das bei der Reaktion der Isocyanatgruppen mit Wasser gebildete Kohlendioxid erfolgen, die Verwendung von weiteren Treibmitteln ist jedoch ebenfalls möglich. So können prinzipiell auch Treibmittel aus der Klasse der Kohlenwasserstoffe wie $C_3$-$C_6$-Alkane, z.B. Butane, n-Pentan, iso-Pentan, cyclo-Pentan, Hexane o.ä. oder halogenierte Kohlenwasserstoffe wie Dichlormethan, Dichlormonofluormethan, Chlordifluorethane, 1,1-Dichlor-2,2,2-Trifluorethan, 2,2-Dichlor-2-fluorethan, insbesondere chlorfreie Fluorkohlenwasserstoffe wie Difluormethan, Trifluormethan, Difluorethan, 1,1,1,2-Tetrafluorethan, Tetrafluorethan (R 134 oder R 134a), 1,1,1,3,3-Pentafluorpropan (R 245 fa), 1,1,1,3,3,3-Hexafluorpropan (R 256), 1,1,1,3,3-Pentafluorbutan (R 365 mfc), Heptafluorpropan oder auch Schwefelhexafluorid verwendet werden. Auch Gemische dieser Treibmittel sind verwendbar.

Die anschließende Aushärtung erfolgt typischerweise bei Raumtemperatur.

In einer bevorzugten Ausführungsform des erfindungsgemäßen Verfahrens, beträgt der Ethylenoxidanteil von A2 $\geq$ 50 Gew.-%, oder bevorzugt $\geq$ 55 Gew.-%, oder bevorzugt $\geq$ 60 Gew.-%.

In einer bevorzugten Ausführungsform des erfindungsgemäßen Verfahrens weist die Mischung der isocyanathaltigen Komponenten ein molares Verhältnis von Urethangruppen zu Isocyanatgruppen in einem Bereich von 1,0 bis 5,0, oder bevorzugt in einem Bereich von 1,1-4,0, oder bevorzugt in einem Bereich von 1,2 bis 3 auf.

In einer bevorzugten Ausführungsform des erfindungsgemäßen Verfahrens liegt bei der Umsetzung der Komponenten A1) bis A3) zu dem isocyanatfunktionellen Präpolymer A) ein molares Verhältnis von NCO zu OH-Gruppen von < 5, oder bevorzugt von < 4, oder bevorzugt von < 3, oder bevorzugt in einem Bereich von 1 bis 5, oder bevorzugt in einem Bereich von 1,5 bis 4,5 vor.

In einer bevorzugten Ausführungsform des erfindungsgemäßen Verfahrens weist das isocyanatfunktionelles Präpolymer A eine Viskosität $\leq$ 50000 mPas, bevorzugt von $\leq$ 25000, oder bevorzugt von $\leq$ 10000, oder bevorzugt in einem Bereich von 100 bis 20000 mPas auf.

In einer bevorzugten Ausführungsform des erfindungsgemäßen Verfahrens weist mindestens ein Teil der isocyanathaltigen Komponenten eine Isocyanatfunktionalität von > 2, bevorzugt in einem Bereich von 2 bis 6, oder bevorzugt in einem Bereich von 2, 1 bis 5 auf.

In einer bevorzugten Ausführungsform des erfindungsgemäßen Verfahrens weist das Polyalkylenoxid A2) eine OH-Zahl in einem Bereich von 40 bis 450 mg KOH/g, bevorzugt in einem Bereich von 75 bis 400, oder bevorzugt in einem Bereich von 113 bis 300 auf.

Weiterer Gegenstand der vorliegenden Erfindung sind überdies die nach dem erfindungsgemäßen Verfahren hergestellten Polyurethanschäume, sowie die Verwendung der hydrophilen, aliphatischen Polyurethanschäume als Bestandteil einer Wundauflage, eines kosmetischen Artikels oder eines Inkontinenzprodukts.

Die Herstellung der erfindungsgemäßen Polyurethanschäume erfolgt wie bereits oben beschrieben bevorzugt durch Mischen der Komponenten A), hergestellt aus den Komponenten A1), A2) und gegebenenfalls A3), D), gegebenenfalls mit C) und/oder H) in beliebiger Reihenfolge, sowie anschließend mit einer Mischung aus B) und gegebenenfalls D), E), F), G), Aufschäumen der Mischung und Aushärtung, bevorzugt durch chemische Vernetzung. Bevorzugt werden die Komponenten A), C) und H) miteinander vorvermischt. Die gegebenenfalls einzusetzenden Salze E) und gegebenenfalls die Tenside F) werden bevorzugt in Form ihrer wässrigen Lösungen dem Reaktionsgemisch zugesetzt. Bevorzugt sind die erfindungsgemäßen Polyurethanschäume hydrophil und weisen aliphatische Einheiten auf.

Die erfindungsgemäßen Polyurethanschäume bzw. die erfindungsgemäß hergestellten Polyurethanschäume weisen bevorzugt eine poröse, zumindest teilweise offenzellige Struktur mit miteinander kommunizierenden Zellen auf. Die Dichte der Polyurethanschäume liegt dabei bevorzugt bei 50 bis 300 g/L.

Das Absorptionsvermögen gegenüber physiologischer Salzlösung beträgt bei den Polyurethanschäumen bevorzugt 300 bis 4000%, oder bevorzugt von 800 bis 3500%, oder bevorzugt von 1000 bis 3000%, bezogen auf das Trockengewicht des Schaums. Die Messung erfolgt dabei nach folgendem Verfahren: (Bestimmung nach DIN EN 13726-1, Teil 3.2)

Im Vergleich zu anderen hydrophilen Schäumen kann mit den erfindungsgemäßen Polyurethanschäumen auch ohne die Verwendung von superabsorbierenden Polymeren eine sehr hohe Absorption von physiologischer Salzlösung erreicht werden. Selbstverständlich ist die Einarbeitung von Superabsorbern aber auch bei den erfindungsgemäßen

EP 3 737 429 B1

Polyurethanschäumen möglich. Dabei können dieselben Techniken der Einarbeitung von Superabsorbern in die erfindungsgemäßen Polyurethanschäume wie in EP 3235520 für die dort beschriebenen Polyurethanschäume Anwendung finden.

Die Polyurethanschäume weisen eine gute mechanische Festigkeit und hohe Elastizität auf. Typischerweise sind die Werte für die Zugfestigkeit größer als 40 kPa, für die Bruchdehnung größer als 30 % bei einer Dichte im Bereich von 50 bis 300 g/L (Bestimmung jeweils nach den Normen wie später unter Methoden beschrieben).

Nach der Herstellung können die Polyurethanschäume nach an sich bekannten Verfahren zu flächigen Materialien verarbeitet werden, welche dann beispielsweise als Bestandteil einer Wundauflage, eines kosmetischen Artikels oder eines Inkontinenzprodukts eingesetzt werden können. In der Regel werden dazu Blockschäume nach gängigen Methoden auf die gewünschte Dicke geschnitten wodurch flächige Materialien mit einer Dicke von typischerweise von 10 $\mu$m bis 5 cm, bevorzugt von 0,1 mm bis 1 cm, besonders bevorzugt von 0,1 mm bis 6 mm, ganz besonders bevorzugt von 0,2 mm bis 6 mm erhalten werden.

Mit Hilfe geeigneter Gießtechniken können die beschriebenen flächigen Materialien aber auch direkt durch Auftrag und Aufschäumen der erfindungsgemäßen Zusammensetzung auf ein Substrat, z.B. ein gegebenenfalls vorbehandeltes Papier, eine Folie, ein Non-Woven oder Textil, erhalten werden.

In einer bevorzugten Variante wird dazu eine Mischung der Ausgangsmaterialien, wie in der PCT Anmeldung mit der Nummer WO 2011/006608 beschrieben, mittels eines Rakels auf ein Substrat aufgebracht, woraufhin im Anschluss an das Rakeln das Aufschäumen erfolgt. Die Spalthöhe des Rakels liegt im Allgemeinen im Bereich von 0,2 bis 20 mm, bevorzugt von 0,5 bis 5 und ganz besonders bevorzugt von 0,8 bis 2 mm. Die Filmbreite des zu verwendenden Rakels kann dem jeweiligen Verwendungszweck angepasst werden. Beispiele sind Filmbreiten zwischen 10 und 5000 mm, bevorzugt zwischen 20 und 2000 mm.

Bevorzugt ist ein Gießverfahren bei dem während der Polyurethanschaum auf eine Schicht oder ein Substrat gegossen wird, eine weitere Schicht auf die Oberseite des Polyurethanschaums aufgebracht wird, vorzugsweise bevor der Polyurethanschaum getrocknet ist. Ein solcher Prozess ist in der Patentanmeldung mit der Anmeldenummer EP 17156493.3 beschrieben und kann hier ebenfalls Anwendung finden.

Die Polyurethanschäume enthalten in der Regel einen nur geringen mit Wasser extrahierbaren Anteil von maximal 2 Gew.-%, bevorzugt von maximal 1 Gew.-%, d.h. sie enthalten nur sehr geringe Mengen an chemisch nicht gebundenen Bestandteilen.

Die Polyurethanschäume können überdies mit weiteren Materialien beispielsweise auf Basis von Hydrogelen, (semi-) permeablen Folien, Schaumfolien, Beschichtungen, Hydrokolloiden oder anderen Schäumen verklebt, laminiert oder beschichtet werden.

Die erfindungsgemäßen Polyurethanschäume sind besonders zur Herstellung von Wundauflagen geeignet. Dabei können die Polyurethanschäume in direktem oder indirektem Kontakt mit der Wunde sein. Bevorzugt werden die Polyurethanschäume jedoch in direktem Kontakt mit der Wunde eingesetzt, um beispielsweise eine optimale Absorption von Wundflüssigkeit zu gewährleisten.

Die Polyurethanschäume, die als Wundauflage eingesetzt werden, müssen zusätzlich noch in einem weiteren Verfahrensschritt sterilisiert werden. Zur Sterilisation kommen die dem Fachmann an sich bekannten Verfahren zum Einsatz, bei denen eine Sterilisation durch thermische Behandlung, chemische Stoffe wie Ethylenoxid oder Bestrahlung, beispielsweise durch Gammabestrahlung, erfolgt. Die Bestrahlung kann dabei gegebenenfalls unter Schutzgasatmosphäre erfolgen. Die erfindungsgemäßen Polyurethanschäume haben dabei den großen Vorteil, dass sie sich bei Bestrahlung, insbesondere bei Bestrahlung mit Gammastrahlen, nicht verfärben.

Ebenfalls möglich ist die Zugabe, Einarbeitung oder Beschichtung von bzw. mit antimikrobiellen, pharmazeutischen oder biologischen Wirkstoffen oder sonstigen Additiven, welche sich beispielsweise in Bezug auf die Wundheilung und die Vermeidung von Keimbelastungen positiv auswirken.

Ein weiterer Gegenstand der Erfindung betrifft eine Polyurethanpräpolymermischung, enthaltend die folgenden Komponenten:

A) ein Polyurethanpräpolymer, erhältlich aus

A1) niedermolekularen Diisocyanaten einer Molmasse von 140 bis 278 g/mol, mit

A2) Polyalkylenoxiden mit einer OH-Funktionalität von zwei oder mehr, bevorzugt in einem Bereich von 2 bis 6, oder bevorzugt in einem Bereich von 2,1 bis 5,

A3) optional weitere isocyanatreaktive Komponenten, die nicht unter A2) fallen;

C) optional heterocyclische, 4-Ring- oder 6-Ring-Oligomere von niedermolekularen Diisocyanaten mit einer Molmasse von 140 bis 278 g/mol, bevorzugt mit einer Isocyanatfunktionalität von 2 bis 6, oder einer Isocyanatfunktionalität von 2,1 bis 5;

D) optional Katalysatoren;

H) optional hydrophile Polyisocyanate erhältlich durch Umsetzung von

H1) niedermolekularen Diisocyanaten einer Molmasse von 140 bis 278 g/mol und / oder daraus herstellbaren Polyisocyanaten mit einer Isocyanatfunktionalität von 2 bis 6, oder einer Isocyanatfunktionalität von 2,1 bis 5;

H2) monofunktionellen Polyalkylenoxiden einer OH-Zahl von 10 bis 250, oder bevorzugt von 20 bis 200 und einem Ethylenoxidanteil von 50 bis 100 mol%, bezogen auf die Gesamtmenge der enthaltenen Oxyalkylengruppen,

wobei die Polyurethanpräpolymermischung, insbesondere die Komponenten A, C und H, einen Isocyanatgehalt in einem Bereich von 2 bis 8 Gew.-%, oder bevorzugt in einem Bereich von 3 bis 7, oder bevorzugt in einem Bereich von 4 bis 6,5 Gew.-% und einen Gehalt von Urethangruppen in einem Bereich von 1,0 bis 3,5 mol/kg, oder bevorzugt in einem Bereich von 1,5 bis 3,0 mol/kg, oder bevorzugt in einem Bereich von 1,7 bis 2,8 mol/kg, jeweils bezogen auf die Gesamtmenge der Polyurethanpräpolymermischung aufweist.

Die erfindungsgemäße Polyurethanpräpolymermischung wird bevorzugt wie im zuvor beschriebenen Verfahren zur Herstellung eines Polyurethanschaums zu dem Polyurethanschaum umgesetzt. Alle für die Polyurethanpräpolymermischung genannten Komponenten weisen die gleichen Eigenschaften auf, wie bereits für diese Komponenten im Zusammenhang mit dem erfindungsgemäßen Verfahren beschrieben.

Ein weiterer Gegenstand der Erfindung betrifft eine Verwendung der erfindungsgemäßen Polyurethanpräpolymermischung oder des erfindungsgemäßen Polyurethanschaums zur Herstellung einer Wundauflage, eines kosmetischen Artikels oder eines Inkontinenzproduktes.

Bevorzugt weist der für die Herstellung der Wundauflage, des kosmetischen Artikels oder des Inkontinenzproduktes verwendete Polyurethanschaum mindestens drei der folgenden Eigenschaften auf:

a) einen Quotient aus Bruchspannung und F20-Wert von mindestens 3,5, bevorzugt von mindestens 4, oder bevorzugt von mindestens 5; oder bevorzugt in einem Bereich von 3,5 bis 30, oder bevorzugt in einem Bereich von 4 bis 30, oder bevorzugt in einem Bereich von 5 bis 30, oder bevorzugt in einem Bereich von 7,5 bis 25;

b) eine Flüssigkeitsabsorption von $\geq$ 300%, oder bevorzugt von $\geq$ 500%, oder bevorzugt von $\geq$ 1000%, oder bevorzugt in einem Bereich von 300 bis 3000 %, oder bevorzugt in einem Bereich von 400 bis 2500%;

c) eine Dichte in einem Bereich von 50 bis 300 g/L, oder bevorzugt in einem Bereich von 60 bis 200 g/L;

d) eine Bruchspannung von mindestens 50 kPa, oder bevorzugt von mindestens 100, oder bevorzugt von mindestens 120; oder bevorzugt in einem Bereich von 50 bis 500, oder bevorzugt in einem Bereich von 100 bis 400;

e) einen F20-Wert von höchstens 50 kPa, oder bevorzugt von höchstens 45 kPa, oder bevorzugt von höchstens 40 kPa, oder bevorzugt von höchstens 20 kPa; oder bevorzugt in einem Bereich von 1 bis 50 kPa, oder bevorzugt in einem Bereich von 2 bis 40 kPa, oder bevorzugt in einem Bereich von 2 bis 20 kPa.

Bevorzugt weist der Polyurethanschaum die Merkmalskombination a) + b) + c) oder a) + b) + d) oder a) + b) + e) oder a) + c) + d) oder a) + c) + e) oder a) + d) + e) oder b) + c) + d) oder b) + c) + e) oder c) + d) + e) oder a) + b) + c) + d), oder a) + b) + c) + e) oder a) + c) + d) + e) oder a) + b) + c) + d) + e) auf.

Ein weiterer Gegenstand der Erfindung betrifft einen Polyurethanschaum aufweisend mindestens die folgenden Eigenschaften:

a) einen Quotient aus Bruchspannung und F20-Wert von mindestens 3,5, bevorzugt von mindestens 4, oder bevorzugt von mindestens 5; oder bevorzugt in einem Bereich von 3,5 bis 30, oder bevorzugt in einem Bereich von 4 bis 29, oder bevorzugt in einem Bereich von 5 bis 28; oder bevorzugt in einem Bereich von 7,5 bis 25;

b) eine Flüssigkeitsabsorption von $\geq$ 300%, oder bevorzugt von $\geq$ 500%, oder bevorzugt von $\geq$ 1000%, oder bevorzugt in einem Bereich von 300 bis 3000 %, oder bevorzugt in einem Bereich von 400 bis 2500%;

c) eine Dichte in einem Bereich von 50 bis 300 g/L, oder bevorzugt in einem Bereich von 60 bis 200 g/L;

d) eine Bruchspannung von mindestens 50 kPa, oder bevorzugt von mindestens 100, oder bevorzugt von mindestens 120; oder bevorzug in einem Bereich von 50 bis 500, oder bevorzugt in einem Bereich von 100 bis 400;

e) einen F20-Wert von höchstens 50 kPa, oder bevorzugt von höchstens 45 kPa, oder bevorzugt von höchstens 40 kPa, oder bevorzugt von höchstens 20 kPa; oder bevorzugt in einem Bereich von 1 bis 100 kPa, oder bevorzugt in einem Bereich von 2 bis 40 kPa, oder bevorzugt in einem Bereich von 2 bis 20 kPa.

Ein weiterer Gegenstand der Erfindung betrifft eine Wundauflage, einen kosmetischen Artikel oder ein Inkontinenzprodukt erhältlich unter Verwendung von erfindungsgemäßen Polyurethanschäumen, oder erfindungsgemäß hergestellten Polyurethanschäumen.

## Methoden

Sofern nicht abweichend gekennzeichnet, beziehen sich alle Prozentangaben auf das Gewicht.
Die Bestimmung der Viskosität erfolgte bei 23 °C und wurde nach DIN 53019 durchgeführt.
Die NCO-Gehalte wurden volumetrisch gemäß DIN-EN ISO 11909 bestimmt.
Die Flüssigkeitsabsorption wurde gemäß DIN EN 13726-1:2002 bestimmt. Die aufgenommene Flüssigkeit wird in % des Trockengewichtes des Schaumes angegeben, wobei das Trockengewicht des Schaumes 100% entspricht, entsprechend:

$$Flüssigkeitsabsorption\ in\ \% = 100 \cdot \frac{Masse\ nach\ Absorption - Masse\ vor\ Absorption(Trockengewicht)}{Masse\ vor\ Absorption}$$

Die angegebenen Werte für die Bruchspannung, die Bruchdehnung und die Spannung bei 20% Dehnung (auch bezeichnet als F20-Wert) wurden gemäß DIN EN ISO 527-2 bestimmt. Die Bruchspannung ist die Kraft pro Querschnittsfläche des Normkörpers, die aufgebracht werden muss, um unter den Bedingungen der Norm einen Bruch, also ein Reißen des Materials des Normkörpers zu provozieren. Die Bruchdehnung gibt die Dehnung des Materials des Normkörpers kurz vor dem Bruch bezogen auf die ursprüngliche Länge des Normkörpers an. Die 20% Dehnung bzw. der F20-Wert gibt an, wie viel Kraft pro Querschnittsfläche des Normkörpers bei der Dehnung des Materials des Normkörpers aufgebracht werden muss, um den Normkörper um 20% seiner ursprünglichen Länge zu dehnen.

## Verwendete Substanzen und Abkürzungen:

Desmodur® N 3300: Aliphatisches Polyisocyanat (HDI-Isocyanurat), NCO-Gehalt 21,8 %, Covestro AG, Leverkusen, Deutschland;
Baymedix® FP520: Hydrophiliertes aliphatisches Polyisocyanat (hydrophiliertes HDI-Isocyanurat), NCO-Gehalt 17,4%, (Urethangehalt 0,4 mol/kg) Covestro AG, Leverkusen, Deutschland.

## Bestimmung des Urethangehalts

Für vorliegende Erfindung kann der Urethangehalt aus der Stöchiometrie der urethanbildenden Reaktion zwischen Hydroxygruppen und Isocyanatgruppen berechnet werden. Da in allen Beispielen die Hydroxygruppen vollständig zu Urethangruppen umgesetzt werden, kann folgende Formel zur Berechnung verwendet werden:

$$Urethangehalt = \frac{Gew.\%\ Hydroxykomponente \cdot OHZ}{5610\ \frac{kg}{mol}}$$

wobei OHZ Hydroxyzahl der eingesetzen Hydroxykomponente bzw. dem entsprechen Durchschnittswert mehrerer Komponenten bedeutet. Beispiele für mögliche Hydroxykomponenten sind mono- oder polyfunktionelle Alkohole oder Polyole. Konkrete Beispiele werden unter A1), A3), G) oder H2) beschrieben.
Bei anschließender Entfernung von urethanfreien Komponenten aus dem Produktgemisch, z.B. durch einen Destillationsprozess, erhöht sich der Polyolgehalt und damit auch der Urethangehalt der Produktmischung.

Rechenbeispiel anhand Beispiel 4

Urethangehalt vor Destillation:

$$Urethangehalt = \frac{63,71 \cdot 190}{5610\frac{kg}{mol}} = 2,16\ \frac{mol}{kg}$$

Urethangehalt nach Destillation:

$$Urethangehalt = \frac{70,07 \cdot 190}{5610\frac{kg}{mol}} = 2,37\ \frac{mol}{kg}$$

Es ist für den Fachmann ersichtlich, dass die hier genutzte Formel modifiziert oder erweitert werden muss, wenn beispielsweise Reaktanden verwendet werden, die bereits Urethangruppen enthalten, andere urethanbildende Reaktionen eingesetzt werden oder konkurrierende Reaktionen auftreten können (z.B. Harnstoffbildung bei Anwesenheit von Aminen).

**Dickenmessung:**

Die Schichtdickenbestimmung wurde mit einem Drucklufttaster und zur Ausgabe der Schichtdicke angeschlossenem Display der Firma Heidehain (MT25P) ermittelt.

**Dichtemessung:**

Zur Dichtebestimmung wurde ein Probenstück mit Hilfe eines Stanzeisens in der Dimension 5 x 5 cm$^2$ (mit abgerundeten Ecken und Kurvenradius von 3 mm) ausgestanzt. Die Höhe wurde aus dem Mittelwert einer 5fach-Bestimmung mittels oben beschriebener Methode ermittelt. Zur anschließenden Berechnung der Dichte wurde die Masse des Probenstückes an einer Mettler Toledo XS603S Waage bestimmt.

**Beispiele**

Beispiel 1 (Erfindungs gemäß)

Zu einem Gemisch aus 1680 g HDI und 5.0 g Dibutylphosphat wurde bei 80 °C innerhalb von 30 min 2960 g eines Polyalkylenoxids mit einer Molmasse von 591 g/mol (OH-Zahl 190 mgKOH/g), gestartet auf 1,3-Propylenglycol und einem Ethylenoxidgewichtsanteil von 87% zugetropft und nachgerührt bis ein NCO-Gehalt von 9,1% erreicht wurde (3,5 h). Das überschüssige HDI wurde durch Dünnschichtdestillation bei 140 °C und 0,7 mbar entfernt. Das Gemisch hatte einen NCO-Gehalt von 5,0 % und einer Viskosität von 5140 mPas. Der berechnete Urethangehalt beträgt 2,4 mol/kg.

Beispiel 2 (Erfindungsgemäß)

Zu einem Gemisch aus 662 g HDI und 1.8 g Dibutylphosphat wurde bei 80 °C innerhalb von 30 min 1167 g eines Polyalkylenoxids mit einer Molmasse von 591 g/mol (OH-Zahl 190 mgKOH/g), gestartet auf 1,3-Propylenglycol und einem Ethylenoxidgewichtsanteil von 87% zugetropft und nachgerührt bis ein NCO-Gehalt von 9,1% erreicht wurde (3,5 h). Das überschüssige HDI wurde durch Dünnschichtdestillation bei 140 °C und 0,4 mbar entfernt. Man erhielt ein Präpolymer mit einem NCO-Gehalt von 5,0 % und einer Viskosität von 4500 mPas. Der berechnete Urethangehalt beträgt 2,4 mol/kg.

Anschließend wurden 5% Desmodur N 3300 hinzugegeben. Das Gemisch hatte einen NCO-Gehalt von 5,7% und eine Viskosität von 3700 mPas. Der berechnete Urethangehalt beträgt 2,3 mol/kg.

Beispiel 3 (Erfindungsgemäß)

Zu einem Gemisch aus 531 g HDI und 1.6 g Dibutylphosphat wurde bei 80 °C innerhalb von 30 min 1169 g eines Polyalkylenoxids mit einer Molmasse von 591 g/mol (OH-Zahl 190 mgKOH/g), gestartet auf 1,3-Propylenglycol und einem Ethylenoxidgewichtsanteil von 87% zugetropft und nachgerührt bis ein NCO-Gehalt von 5,8% erreicht wurde (3,5 h). Das überschüssige HDI wurde durch Dünnschichtdestillation bei 140 °C und 0,6 mbar entfernt. Man erhielt ein Präpolymer mit einem NCO-Gehalt von 3,7 % und einer Viskosität von 11500 mPas. Der berechnete Urethangehalt beträgt 2,4 mol/kg. Anschließend wurden 5% Baymedix® FP520 hinzugegeben. Das Gemisch hatte einen NCO-Gehalt von 4,1% und eine Viskosität von 15700 mPas. Der berechnete Urethangehalt beträgt 2,3 mol/kg.

Beispiel 4 (Erfindungsgemäß)

Zu einem Gemisch aus 541 g HDI und 1.5 g Dibutylphosphat wurde bei 80 °C innerhalb von 30 min 1059 g eines Polyalkylenoxids mit einer Molmasse von 591 g/mol (OH-Zahl 190 mgKOH/g), gestartet auf 1,3-Propylenglycol und einem Ethylenoxidgewichtsanteil von 87% zugetropft und nachgerührt bis ein NCO-Gehalt von 7,3% erreicht wurde (3,5 h). Das überschüssige HDI wurde durch Dünnschichtdestillation bei 140 °C und 0,6 mbar entfernt. Man erhielt ein Präpolymer mit einem NCO-Gehalt von 4,5 % und einer Viskosität von 6690 mPas. Der berechnete Urethangehalt beträgt 2,4 mol/kg. Anschließend wurden 5% Baymedix® FP520 hinzugegeben. Das Gemisch hatte einen NCO-Gehalt von 4,7% und eine Viskosität von 9800 mPas. Der berechnete Urethangehalt beträgt 2,3 mol/kg.

Beispiel 5 (Erfindungsgemäß)

Zu einem Gemisch aus 662 g HDI und 1.8 g Dibutylphosphat wurde bei 80 °C innerhalb von 30 min 1167 g eines Polyalkylenoxids mit einer Molmasse von 591 g/mol (OH-Zahl 190 mgKOH/g), gestartet auf 1,3-Propylenglycol und einem Ethylenoxidgewichtsanteil von 87% zugetropft und nachgerührt bis ein NCO-Gehalt von 9,1% erreicht wurde (3,5 h). Das überschüssige HDI wurde durch Dünnschichtdestillation bei 140 °C und 0,4 mbar entfernt. Man erhielt ein Präpolymer mit einem NCO-Gehalt von 5,0 % und einer Viskosität von 4500 mPas. Der berechnete Urethangehalt beträgt 2,4 mol/kg. Anschließend wurden 5% Baymedix® FP520 hinzugegeben. Das Gemisch hatte einen NCO-Gehalt von 5,2% und eine Viskosität von 7030 mPas. Der berechnete Urethangehalt beträgt 2,3 mol/kg.

Beispiel 6 (Erfindungsgemäß)

Zu einem Gemisch aus 4616 g HDI und 12 g Dibutylphosphat wurde bei 80 °C innerhalb von 30 min 7384 g eines Polyalkylenoxids mit einer Molmasse von 591 g/mol (OH-Zahl 190 mgKOH/g), gestartet auf 1,3-Propylenglycol und einem Ethylenoxidgewichtsanteil von 87% zugetropft und nachgerührt bis ein NCO-Gehalt von 10,4% erreicht wurde (3,5 h). Das überschüssige HDI wurde durch Dünnschichtdestillation bei 140 °C und 0,3 mbar entfernt. Man erhielt ein Präpolymer mit einem NCO-Gehalt von 5,5 % und einer Viskosität von 3450 mPas. Der berechnete Urethangehalt beträgt 2,4 mol/kg. Anschließend wurden 5% Baymedix® FP520 hinzugegeben. Das Gemisch hatte einen NCO-Gehalt von 5,9% und eine Viskosität von 4100 mPas. Der berechnete Urethangehalt beträgt 2,3 mol/kg.

Beispiel 7 (Erfindungsgemäß)

Zu einem Gemisch aus 4984 g HDI und 12 g Dibutylphosphat wurde bei 80 °C innerhalb von 30 min 7016 g eines Polyalkylenoxids mit einer Molmasse von 591 g/mol (OH-Zahl 190 mgKOH/g), gestartet auf 1,3-Propylenglycol und einem Ethylenoxidgewichtsanteil von 87% zugetropft und nachgerührt bis ein NCO-Gehalt von 12,5% erreicht wurde (3,5 h). Das überschüssige HDI wurde durch Dünnschichtdestillation bei 140 °C und 0,3 mbar entfernt. Man erhielt ein Präpolymer mit einem NCO-Gehalt von 5,7 % und einer Viskosität von 2670 mPas. Der berechnete Urethangehalt beträgt 2,3 mol/kg. Anschließend wurden 5% Baymedix® FP520 hinzugegeben. Das Gemisch hatte einen NCO-Gehalt von 6,1% und eine Viskosität von 3220 mPas. Der berechnete Urethangehalt beträgt 2,2 mol/kg.

Beispiel 8 (Erfindungsgemäß)

Zu einem Gemisch aus 420 g HDI und 1,6 g Dibutylphosphat wurde bei 80 °C innerhalb von 30 min 1021 g eines Polyalkylenoxids mit einer Molmasse von 591 g/mol (OH-Zahl 190 mgKOH/g), gestartet auf 1,3-Propylenglycol und einem Ethylenoxidgewichtsanteil von 87% zugetropft und nachgerührt bis ein NCO-Gehalt von 23,5% erreicht wurde (3,5 h). Das überschüssige HDI wurde durch Dünnschichtdestillation bei 140 °C und 0,4 mbar entfernt. Man erhielt ein Präpolymer mit einem NCO-Gehalt von 7,5 % und einer Viskosität von 905 mPas. Der berechnete Urethangehalt beträgt 2,2 mol/kg. Anschließend wurden 5% Baymedix® FP520 hinzugegeben. Das Gemisch hatte einen NCO-Gehalt von 8,0% und eine Viskosität von 1440 mPas. Der berechnete Urethangehalt beträgt 2,2 mol/kg.

Beispiel 9 (Erfindungsgemäß)

Zu einem Gemisch aus 710 g HDI und 0,5 g Dibutylphosphat wurde bei 80 °C innerhalb von 30 min 200 g Polyethylenglycol mit einer Molmasse von 400 g/mol (OH-Zahl 281 mgKOH/g), zugetropft und nachgerührt bis ein NCO-Gehalt von 7,5% erreicht wurde (3,5 h). Das überschüssige HDI wurde durch Dünnschichtdestillation bei 140 °C und 0,4 mbar entfernt. Man erhielt ein Präpolymer mit einem NCO-Gehalt von 5,0 % und einer Viskosität von 15900 mPas. Der berechnete Urethangehalt beträgt 3,2 mol/kg. Anschließend wurden 5,0% Baymedix® FP520 hinzugegeben. Das Gemisch hatte einen NCO-Gehalt von 5,1% und eine Viskosität von 23100 mPas. Der berechnete Urethangehalt beträgt 3,0 mol/kg.

Beispiel 10 (Erfindungsgemäß)

Zu einem Gemisch aus 361 g HDI und 2,1 g Dibutylphosphat wurde bei 80 °C innerhalb von 30 min 1216 g eines Polyalkylenoxids mit einer Molmasse von 998 g/mol (OH-Zahl 112 mgKOH/g), gestartet auf 1,3-Propylenglycol und einem Ethylenoxidgewichtsanteil von 89%, zugetropft und nachgerührt bis ein NCO-Gehalt von 15.8% erreicht wurde (3,5 h). Das überschüssige HDI wurde durch Dünnschichtdestillation bei 140 °C und 0,4 mbar entfernt. Man erhielt ein Präpolymer mit einem NCO-Gehalt von 4,9 % und einer Viskosität von 1360 mPas. Der berechnete Urethangehalt beträgt 1,5 mol/kg. Anschließend wurden 5,0% Baymedix® FP520 hinzugegeben. Das Gemisch hatte einen NCO-Gehalt von 5,5% und eine Viskosität von 2020 mPas. Der berechnete Urethangehalt beträgt 1,5 mol/kg.

Beispiel 11 (Nicht Erfindungsgemäß)

Zu einem Gemisch aus 1260 g HDI und 2,0 g Dibutylphosphat wurde bei 80 °C innerhalb von 30 min 1972 g eines Polyalkylenoxids mit einer Molmasse von 3951 g/mol (OH-Zahl 28,4 mgKOH/g), gestartet auf 1,3-Propylenglycol und einem Ethylenoxidgewichtsanteil von 78%, zugetropft und nachgerührt bis ein NCO-Gehalt von 18,1% erreicht wurde (3,5 h). Das überschüssige HDI wurde durch Dünnschichtdestillation bei 140 °C und 0,5 mbar entfernt. Man erhielt ein Präpolymer mit einem NCO-Gehalt von 2,0 % und einer Viskosität von 3690 mPas. Der berechnete Urethangehalt beträgt 0,5 mol/kg. Anschließend wurden 5,0% Baymedix® FP520 hinzugegeben. Das Gemisch hatte einen NCO-Gehalt von 2,6% und eine Viskosität von 4250 mPas. Der berechnete Urethangehalt beträgt 0,5 mol/kg.

Beispiel 12 (nicht erfindungs gemäß)

Die isocyanathaltigen Mischungen von Beispiel 10 und 11 wurden im Verhältnis 1:1 gemischt. Das Gemisch hatte einen NCO-Gehalt von 4,3% und eine Viskosität von 2940 mPas. Der berechnete Urethangehalt beträgt 0,97 mol/kg.

Beispiel 13 (nicht erfindungs gemäß)

Zu einem Gemisch aus 1130 g HDI und 1,78 g Dibutylphosphat wurde bei 80 °C innerhalb von 30 min 652 g Tetraethylenglycol (OH-Zahl 580 mgKOH/g) zugetropft und nachgerührt bis ein NCO-Gehalt von 15,7% erreicht wurde (3,5 h). Der berechnete Urethangehalt beträgt 3,8 mol/kg. Während des Abkühlens auf Raumtemperatur kam es zur Kristallisation, so dass ein weißer Feststoff entstand. Das Material konnte aufgrund des Feststoffcharakters nicht zum Schaum verarbeitet werden.

Beispiel 14 (nicht erfindungsgemäß)

Zu einem Gemisch aus 998 g HDI und 1,9 g Dibutylphosphat wurde bei 80 °C innerhalb von 30 min 873 g Tetraethylenglycol (OH-Zahl 580 mgKOH/g) zugetropft und nachgerührt bis ein NCO-Gehalt von 6,4% erreicht wurde (7 h). Der berechnete Urethangehalt beträgt 4,8 mol/kg. Während des Abkühlens auf Raumtemperatur kam es zur Kristallisation, so dass ein weißer Feststoff entstand. Das Material konnte aufgrund des Feststoffcharakters nicht zum Schaum verarbeitet werden.

Beispiel 15 (nicht erfindungsgemäß)

Zu einem Gemisch aus 1184 g HDI und 2.58 g Dibutylphosphat wurde bei 80 °C innerhalb von 2 h 1395 g eines Polyalkylenoxids mit einer Molmasse von 4680 g/mol (OH-Zahl 36 mgKOH/g), gestartet auf Glycerin, einem Ethylenoxidgewichtsanteil von 72% und einem Propylenoxidgewichtsanteil von 28 % zugetropft und nachgerührt bis ein NCO-Gehalt von 21,4% erreicht wurde (2,5 h). Das überschüssige HDI wurde durch Dünnschichtdestillation bei 140 °C und 0,1 mbar entfernt. Man erhielt ein Präpolymer mit einem NCO-Gehalt von 2,5 % und einer Viskosität von 3500 mPas. Der berechnete Urethangehalt beträgt 0,6 mol/kg.

Beispiel 16 (nicht erfindungsgemäß)

Zu einem Gemisch aus 2805 g HDI und 3.25 g Dibutylphosphat wurde bei 80 °C innerhalb von 30 min 649 g eines Polyalkylenoxids mit einer Molmasse von 591 g/mol (OH-Zahl 190 mgKOH/g), gestartet auf 1,3-Propylenglycol und einem Ethylenoxidgewichtsanteil von 87% zugetropft und nachgerührt bis ein NCO-Gehalt von 37,6% erreicht wurde (1 h). Das überschüssige HDI wurde durch Dünnschichtdestillation bei 140 °C und 0,5 mbar entfernt. Man erhielt ein Präpolymer mit einem NCO-Gehalt von 9,1% und einer Viskosität von 700 mPas. Der berechnete Urethangehalt beträgt 2,2 mol/kg. Anschließend wurden 5,0% Desmodur N 3300 hinzugegeben. Das Gemisch hat einen NCO-Gehalt von 9,3% und der berechnete Urethangehalt beträgt 2,1 mol/kg.

Herstellung von Schaumstoffen aus den Beispielen

Die Präpolymermischungen aus den Beispielen wurden jeweils in einen 500 mL PP Becher der Firma Sarstedt gefüllt und mittels einem Rührer der Firma Pendraulik (Disperlux green 037) für 15 Sekunden mit einer Drehzahl von 930 Upm homogenisiert. Anschließend wurde eine definierte Menge (Tabelle 1) zugegeben. Die Wasserphase bestand aus 93,5% Wasser, 1,3% Natriumhydrogencarbonat, 0,4% Zitronensäuremonohydrat und 4,8% Pluronic PE6800. Dann wurde weitere 7 Sekunden gerührt. Das Gemisch wurde mit Hilfe eines Rakels (Spalthöhe 1500 $\mu$m) auf ein Trennpapier der Firma Felix Schoeller (Y05200) aufgetragen. Nach 60 Sekunden (bezogen auf den Versuchsstart) wurde ein genadeltes Trennpapier der Firma Felix Schoeller (Y05200) aufgelegt. Der entstandene Schaum wurde über Nacht bei Raumtemperatur zum Trocknen liegen gelassen.

**Tabelle 1. Eigenschaften von Schäumen hergestellt aus den Beispielen.**

| Beispiel | NCO-Gehalt der isocyana thaltigen Mischun g [%] * | Ureth angeh alt [mol/k g] | Massenverh ältnis der isocyanathaltigen Mischung* zu wässriger Phase | Dichte [g/L] | Bruchspannung [kPa] | Bruchdehnung [%] | F20 [kPa] | Bruchspannung /F20 | Absorption [%] |
|---|---|---|---|---|---|---|---|---|---|
| 1 | 5,0 | 2,4 | 5:1 | 113 | 339 | 515 | 46,0 | 7,4 | 1818 |
| 2 | 5,7 | 2,3 | 7:1 | 88 | 147 | 437 | 9,3 | 15,8 | 1762 |
| 3 | 4,1 | 2,3 | 5:1 | 120 | 170 | 456 | 141 | 12.1 | 1462 |
| 4 | 4,7 | 2,3 | 5:1 | 106 | 167 | 577 | 11,7 | 14,3 | 1751 |
| 5 | 5,2 | 2,3 | 5:1 | 96 | 131 | 476 | 9,8 | 13,4 | 1824 |
| 6 | 5,9 | 2,3 | 5:1 | 85 | 92 | 338 | 12,9 | 7,1 | 1382 |
| 7 | 6,1 | 2,2 | 5:1 | 76 | 134 | 288 | 22,6 | 5,9 | 922 |
| 8 | 8,0 | 2,2 | 4:1 | 61 | 68 | 165 | 17,2 | 4,0 | 738 |
| 9 | 5,1 | 3,0 | 5:1 | 89 | 140 | 277 | 21,6 | 6,5 | 858 |
| 10 | 5,5 | 1,5 | 7:1 | 85 | 106 | 458 | 8,7 | 12,2 | 2600 |
| 11 | 2,6 | 0,5 | 5:1 | 492 | 519 | 564 | 78,0 | 6,7 | 961 |
| 12 | 4,3 | 0,97 | 5:1 | 401 | 495 | 475 | 67,6 | 7,3 | 866 |
| 13 | 15,7 | 3,8 | n.b. | n.b. | n.b. | n.b. | n.b. | n.b. | n.b. |
| 14 | 6,4 | 4,8 | n.b. | n.b. | n.b. | n.b. | n.b. | n.b. | n.b. |
| 15 | 2,5 | 0,6 | 5:1 | 316 | 155 | 46 | 80 | 1,9 | 724 |
| 16 | 9,3 | 2,1 | 5:1 | 57 | 34 | 59 | 18,7 | 1,8 | 445 |

*die isocyanathaltige Mischung bezieht sich auf die Summe aller isocyanathaltigen Komponenten, insbesondere Komponenten A), C) und H)
** Die Beispiele 13 und 14 konnten aufgrund ihres Feststoffcharakters nicht zum Schaum verarbeitet werden.

Wie bereits in der Einleitung der Anmeldung erwähnt, ist es wünschenswert einen Schaum bereitzustellen, der beispielsweise zur Herstellung einer Wundauflage geeignet ist, der optimierte Eigenschaften aufweist. Zu diesen optimierten Eigenschaften zählt eine möglichst hohe Flexibilität bei einer hohen Bruchdehnung und möglichst hoher Absorptionsfähigkeit. Anhand der Werte in Tabelle 1 ist zu erkennen, dass die F20-Werte der erfindungsgemäßen Beispiele alle unterhalb von 50 kPa liegen bei einem Quotienten aus Bruchspannung und dem F20-Werte von größer 3,5. Dahingegen weisen die nicht-erfindungsgemäßen Beispiele in keinem Fall eine solche Kombination auf und sind daher deutlich unflexibler und/oder unzureichend reißfest und somit ungeeignet für die Anwendung als Wundauflage. Außerdem war es möglich aus den erfindungsgemäßen isocyanathaltigen Beispielen einfach Schäume im gewünschten Dichtebereich von 50 bis 300 g/L herzustellen, insbesondere im bevorzugten Bereich von 60 bis 200 g/L.

**Patentansprüche**

1. Ein Verfahren zur Herstellung von Polyurethanschäumen, bei dem Zusammensetzungen umfassend

   A) isocyanatfunktionelle Präpolymere erhältlich durch die Umsetzung von

   A1) niedermolekularen Diisocyanaten einer Molmasse von 140 bis 278 g/mol, mit
   A2) Polyalkylenoxiden mit einer OH-Funktionalität von zwei oder mehr,
   A3) optional weitere isocyanatreaktive Komponenten, die nicht unter A2) fallen;

   B) Wasser in einer Menge von mindestens 2 Gew.-%, bezogen auf das Gesamtgewicht der Zusammensetzung;
   C) optional heterocyclische, 4-Ring- oder 6-Ring-Oligomere von niedermolekularen Diisocyanaten mit einer Molmasse von 140 bis 278 g/mol,
   D) optional Katalysatoren;
   E) optional Salze schwacher Säuren deren korrespondierende freie Säuren in Wasser bei 25 °C einen pKS-Wert von $\geq 3{,}0$ und $\leq 14{,}0$ aufweisen;
   F) optional Tenside; und
   G) optional ein- oder mehrwertige Alkohole oder Polyole;
   H) optional hydrophile Polyisocyanate erhältlich durch Umsetzung von

   H1) niedermolekularen Diisocyanaten einer Molmasse von 140 bis 278 g/mol und / oder daraus herstellbaren Polyisocyanaten mit einer Isocyanatfunktionalität von 2 bis 6, mit
   H2) monofunktionellen Polyalkylenoxiden einer OH-Zahl von 10 bis 250, und einem Ethylenoxidanteil von 50 bis 100 mol% bezogen auf die Gesamtmenge der enthaltenen Oxyalkylengruppen.

   bereitgestellt, aufgeschäumt und ausgehärtet werden,

   wobei die isocyanathaltigen Komponenten, insbesondere die Komponenten A, C und H, insgesamt einen volumetrisch gemäß DIN-EN ISO 11909 bestimmten Isocyanatgehalt in einem Bereich von 2 bis 8 Gew.-% und einen in Übereinstimmung mit dem in der Beschreibung angegebenen Verfahren bestimmten Gehalt an Urethangruppen von 1,0 bis 3,5 mol/kg, jeweils bezogen auf die Gesamtmenge der isocyanathaltigen Komponenten, aufweisen.

2. Das Verfahren gemäß Anspruch 1, **dadurch gekennzeichnet, dass** die Komponente A) einen Gewichtsanteil an niedermolekularen Diisocyanaten mit einer Molmasse von 140 bis 278 g/mol von unter 1,0 Gew.-% bezogen auf das Präpolymer aufweist.

3. Das Verfahren gemäß einem der voranstehenden Ansprüche, **dadurch gekennzeichnet, dass** aliphatische Isocyanate als Komponente A1) eingesetzt werden.

4. Das Verfahren gemäß einem der vorangehenden Ansprüche, **dadurch gekennzeichnet, dass** folgende Schritte durchgeführt werden:

   I) Herstellen des Präpolymers A) aus den Komponenten A1), A2) und gegebenenfalls A3), D),
   II) Optionales Mischen der Komponenten A), C) und H) sowie anderer isocyanathaltiger Komponenten unter Erhalt einer Präpolymer-Mischung,
   III) Optional Zugabe von A3) und gegebenenfalls D),
   IV) Optionales Mischen der Komponente B) mit allen weiteren Komponenten, insbesondere D), E), F) und G) außer der Präpolymer-Mischung,
V) Mischen der unter I) bis III) erhaltenen Präpolymer-Mischung mit der Mischung aus IV).

5. Das Verfahren gemäß einem der voranstehenden Ansprüche, **dadurch gekennzeichnet, dass** der Ethylenoxidanteil von A2 ≥ 50 Gew.-% beträgt.

6. Das Verfahren gemäß einem der voranstehenden Ansprüche, **dadurch gekennzeichnet, dass** die Mischung der isocyanathaltigen Komponenten ein molares Verhältnis von Urethangruppen zu Isocyanatgruppen von 1,0 bis 5,0 aufweist.

7. Das Verfahren gemäß einem der voranstehenden Ansprüche, **dadurch gekennzeichnet, dass** bei der Umsetzung der Komponenten A1) bis A3) zu dem isocyanatfunktionellen Präpolymer A) ein molares Verhältnis von NCO zu OH-Gruppen von < 5 vorliegt.

8. Das Verfahren gemäß einem der voranstehenden Ansprüche, **dadurch gekennzeichnet, dass** das isocyanatfunktionelles Präpolymer A eine Viskosität ≤ 50000 mPas aufweist.

9. Das Verfahren gemäß einem der voranstehenden Ansprüche, **dadurch gekennzeichnet, dass** mindestens ein Teil der isocyanathaltigen Komponenten eine Isocyanatfunktionalität von > 2 aufweisen.

10. Das Verfahren gemäß einem der voranstehenden Ansprüche, **dadurch gekennzeichnet, dass** das Polyalkylenoxid A2) eine OH-Zahl in einem Bereich von 40 bis 450 mg KOH/g aufweist.

11. Ein Polyurethanschaum erhältlich gemäß einem Verfahren gemäß einem der voranstehenden Ansprüche.

12. Eine Polyurethanpräpolymermischung, enthaltend die folgenden Komponenten:

    A) ein Polyurethanpräpolymer, erhältlich aus

        A1) niedermolekularen Diisocyanaten einer Molmasse von 140 bis 278 g/mol, mit
        A2) Polyalkylenoxiden mit einer OH-Funktionalität von zwei oder mehr,
        A3) optional weitere isocyanatreaktive Komponenten, die nicht unter A2) fallen,

    C) optional heterocyclische, 4-Ring- oder 6-Ring-Oligomere von niedermolekularen Diisocyanaten mit einer Molmasse von 140 bis 278 g/mol,
    D) optional Katalysatoren;
    H) optional hydrophile Polyisocyanate erhältlich durch Umsetzung von

        H1) niedermolekularen Diisocyanaten einer Molmasse von 140 bis 278 g/mol und / oder daraus herstellbaren Polyisocyanaten mit einer Isocyanatfunktionalität von 2 bis 6, mit
        H2) monofunktionellen Polyalkylenoxiden einer OH-Zahl von 10 bis 250, und einem Ethylenoxidanteil von 50 bis 100 mol% bezogen auf die Gesamtmenge der enthaltenen Oxyalkylengruppen,

    wobei die Polyurethanpräpolymermischung, insbesondere die Komponenten A, C und H, einen Isocyanatgehalt in einem Bereich von 2 bis 8 Gew.-% und einen Gehalt von Urethangruppen von 1,0 bis 3,5 mol/kg, jeweils bezogen auf die Gesamtmenge der Polyurethanpräpolymermischung aufweist.

13. Verwendung einer Polyurethanpräpolymermischung gemäß Anspruch 12 oder eines Polyurethanschaums nach Anspruch 11 zur Herstellung einer Wundauflage, eines kosmetischen Artikels oder eines Inkontinenzproduktes.

14. Ein Polyurethanschaum gemäß Anspruch 11 aufweisend mindestens die folgenden Eigenschaften:

    a) Einem Quotient aus gemäß DIN EN ISO 527-2 bestimmter Bruchspannung und gemäß DIN EN ISO 527-2 bestimmter Spannung bei 20% Dehnung (F20-Wert) von mindestens 3,5;
    b) Eine gemäß DIN EN 13726-1:2002 bestimmte und als Prozent des Trockengewichtes angegebene Flüssigkeitsabsorption ≥ 300%;
    c) Eine in Übereinstimmung mit dem in der Beschreibung angegebenen Verfahren bestimmte Dichte zwischen 50 und 300 g/L;

d) Eine gemäß DIN EN ISO 527-2 bestimmte Bruchspannung von mindestens 50 kPa;

e) Einen gemäß DIN EN ISO 527-2 bestimmten F20-Wert von höchstens 50 kPa.

15. Eine Wundauflage, ein kosmetischer Artikel oder ein Inkontinenzprodukt erhältlich unter Verwendung von Polyurethanschäumen gemäß einem der Ansprüche 11 oder 14, oder hergestellt gemäß einem der Ansprüche 1 bis 10.

**Claims**

1. A process for producing polyurethane foams, in which compositions comprising

    A) isocyanate-functional prepolymers obtainable by the reaction of

        A1) low molecular weight diisocyanates of molar mass from 140 to 278 g/mol with
        A2) polyalkylene oxides having an OH functionality of two or more,
        A3) optionally further isocyanate-reactive components not covered by A2);

    B) water in an amount of at least 2% by weight, based on the total weight of the composition;
    C) optionally heterocyclic 4-membered or 6-membered ring oligomers of low molecular weight diisocyanates having a molar mass of 140 to 278 g/mol,
    D) optionally catalysts;
    E) optionally salts of weak acids, the corresponding free acids of which have a pKA in water at 25°C of $\geq$ 3.0 and $\leq$ 14.0;
    F) optionally surfactants; and
    G) optionally mono- or polyhydric alcohols or polyols;
    H) optionally hydrophilic polyisocyanates obtainable by reaction of

        H1) low molecular weight diisocyanates of molar mass from 140 to 278 g/mol and/or polyisocyanates preparable therefrom and having an isocyanate functionality of 2 to 6 with
        H2) monofunctional polyalkylene oxides of OH number from 10 to 250 and of ethylene oxide content from 50 to 100 mol%, based on the total amount of the oxyalkylene groups present,

    are provided, foamed and cured,

    wherein the isocyanate-containing components, especially components A, C and H, have a total isocyanate content, determined by volumetric means according to DIN-EN ISO 11909, within a range from 2% to 8% by weight and a content of urethane groups, determined by the method specified in the description, of 1.0 to 3.5 mol/kg, based in each case on the total amount of the isocyanate-containing components.

2. The process according to Claim 1, **characterized in that** component A) has a proportion by weight of low molecular weight diisocyanates having a molar mass of 140 to 278 g/mol of below 1.0% by weight, based on the prepolymer.

3. The process according to either of the preceding claims, **characterized in that** aliphatic isocyanates are used as component A1).

4. The process according to any of the preceding claims, **characterized in that** the following steps are conducted:

    I) preparing the prepolymer A) from components A1), A2) and optionally A3), D),
    II) optionally mixing components A), C) and H) and other isocyanate-containing components to obtain a prepolymer mixture,
    III) optionally adding A3) and optionally D),
    IV) optionally mixing component B) with all other components, especially D), E), F) and G), apart from the prepolymer mixture,
    V) mixing the prepolymer mixture obtained in I) to III) with the mixture from IV).

5. The process according to any of the preceding claims, **characterized in that** the ethylene oxide content of A2) is $\geq$ 50% by weight.

**6.** The process according to any of the preceding claims, **characterized in that** the mixture of isocyanate-containing components has a molar ratio of urethane groups to isocyanate groups of 1.0 to 5.0.

**7.** The process according to any of the preceding claims, **characterized in that** there is a molar ratio of NCO to OH groups in the reaction of components A1) to A3) to give the isocyanate-functional prepolymer A) of $\leq$ 5.

**8.** The process according to any of the preceding claims, **characterized in that** the isocyanate-functional prepolymer A has a viscosity of $\leq$ 50 000 mPas.

**9.** The process according to any of the preceding claims, **characterized in that** at least some of the isocyanate-containing components have an isocyanate functionality of > 2.

**10.** The process according to any of the preceding claims, **characterized in that** the polyalkylene oxide A2) has an OH number within a range from 40 to 450 mg KOH/g.

**11.** A polyurethane foam obtainable by a process according to any of the preceding claims.

**12.** A polyurethane prepolymer mixture comprising the following components:

A)a polyurethane prepolymer obtainable from

A1) low molecular weight diisocyanates of molar mass from 140 to 278 g/mol with
A2) polyalkylene oxides having an OH functionality of two or more,
A3) optionally further isocyanate-reactive components not covered by A2),

C) optionally heterocyclic 4-membered or 6-membered ring oligomers of low molecular weight diisocyanates having a molar mass of 140 to 278 g/mol,
D) optionally catalysts;
H) optionally hydrophilic polyisocyanates obtainable by reaction of

H1) low molecular weight diisocyanates of molar mass from 140 to 278 g/mol and/or polyisocyanates preparable therefrom and having an isocyanate functionality of 2 to 6 with
H2) monofunctional polyalkylene oxides of OH number from 10 to 250 and of ethylene oxide content from 50 to 100 mol%, based on the total amount of the oxyalkylene groups present,

wherein the polyurethane prepolymer mixture, especially components A, C and H, has an isocyanate content within a range from 2% to 8% by weight and a content of urethane groups of 1.0 to 3.5 mol/kg, based in each case on the total amount of the polyurethane prepolymer mixture.

**13.** Use of a polyurethane prepolymer mixture according to Claim 12 or of a polyurethane foam according to Claim 11 for production of a wound dressing, a cosmetic article or an incontinence product.

**14.** A polyurethane foam according to Claim 11 having at least the following properties:

a) a quotient of breaking strength, determined to DIN EN ISO 527-2, and stress, determined to DIN EN ISO 527-2, at 20% elongation (F20) of at least 3.5;
b) a liquid absorption, determined to DIN EN 13726-1:2002 and reported as percentage of the dry weight, $\geq$ 300%;
c) a density, determined by the process reported in the description, between 50 and 300 g/l;
d) a breaking strength, determined to DIN EN ISO 527-2, of at least 50 kPa;
e) an F20, determined to DIN EN ISO 527-2, of at most 50 kPa.

**15.** A wound dressing, a cosmetic article or an incontinence product obtainable using polyurethane foams according to any of Claims 11 or 14, or produced according to any of Claims 1 to 10.

**Revendications**

**1.** Procédé pour la production de mousses de polyuréthane, dans lequel sont fournies, expansées et durcies des compositions comprenant

A) des prépolymères à fonction isocyanate pouvant être obtenus par la mise en réaction de

A1) diisocyanates de faible masse moléculaire, ayant une masse molaire de 140 à 278 g/mole, avec
A2) des polyoxyalkylènes ayant une fonctionnalité OH de deux ou plus,
A3) en option des composants supplémentaires réactifs avec des isocyanates, qui ne rentrent pas dans A2) ;

B) de l'eau en une quantité d'au moins 2 % en poids, par rapport au poids total de la composition ;
C) en option des oligomères hétérocycliques à 4 cycles ou 6 cycles de diisocyanates de faible masse moléculaire ayant une masse molaire de 140 à 278 g/mole ;
D) en option des catalyseurs ;
E) en option des sels d'acides faibles dont les acides libres correspondants présentent dans l'eau à 25 °C un pKa de $\geq$ 3,0 et $\leq$ 14,0 ;
F) en option des tensioactifs ; et
G) en option des alcools mono- ou polyhydriques ou des polyols ; et
H) en option des polyisocyanates hydrophiles pouvant être obtenus par mise en réaction de

H1) diisocyanates de faible masse moléculaire ayant une masse molaire de 140 à 278 g/mole et/ou polyisocyanates pouvant être produits à partir de ceux-ci, ayant une fonctionnalité isocyanate de 2 à 6, avec
H2) des polyoxyalkylènes monofonctionnels ayant un indice OH de 10 à 250, et une teneur en oxyde d'éthylène de 50 à 100 % en moles, par rapport à la quantité totale des groupes oxyalkylène contenus,

les composants contenant des isocyanates, en particulier les composants A, C et H, ayant au total une teneur en isocyanates, déterminée par volumétrie selon DIN-EN ISO 11909, dans une plage de 2 à 8 % en poids et une teneur en groupes uréthane, déterminée conformément à la méthode indiquée dans la description, de 1,0 à 3,5 moles/kg, chaque fois par rapport à la quantité totale des composants contenant des isocyanates.

2. Procédé selon la revendication 1, **caractérisé en ce que** le composant A) présente une proportion en poids de diisocyanates de faible masse moléculaire, ayant une masse molaire de 140 à 278 g/mole, inférieure à 1,0 % en poids par rapport au prépolymère.

3. Procédé selon l'une quelconque des revendications précédentes, **caractérisé en ce que** des isocyanates aliphatiques sont utilisés en tant que composant A1).

4. Procédé selon l'une quelconque des revendications précédentes, **caractérisé en ce que** sont effectuées les étapes suivantes :

I) préparation du prépolymère A) à partir des composants A1), A2) et éventuellement A3), D),
II) mélangeage optionnel des composants A), C) et H) ainsi que d'autres composants contenant des isocyanates, avec obtention d'un mélange de prépolymère,
III) addition optionnelle d'A3) et éventuellement de D),
IV) mélangeage optionnel du composant B) avec tous les autres composants, en particulier D), E), F) et G) hormis le mélange de prépolymère,
V) mélangeage du mélange de prépolymère obtenu en I) à III) avec le mélange obtenu en IV).

5. Procédé selon l'une quelconque des revendications précédentes, **caractérisé en ce que** la proportion d'oxyde d'éthylène d'A2 est $\geq$ 50 % en poids.

6. Procédé selon l'une quelconque des revendications précédentes, **caractérisé en ce que** le mélange des composants contenant des isocyanates présente un rapport molaire de groupes uréthane à groupes isocyanate de 1,0 à 5,0.

7. Procédé selon l'une quelconque des revendications précédentes, **caractérisé en ce que** lors de la réaction des composants A1) à A3) conduisant au prépolymère A) à fonction isocyanate, un rapport molaire de NCO à groupes OH de $\leq$ 5 est présent.

8. Procédé selon l'une quelconque des revendications précédentes, **caractérisé en ce que** le prépolymère A à fonction isocyanate présente une viscosité $\leq$ 50 000 mPa.s.

9. Procédé selon l'une quelconque des revendications précédentes, **caractérisé en ce qu'**au moins une partie des composants contenant des isocyanates présentent une fonctionnalité isocyanate de > 2.

10. Procédé selon l'une quelconque des revendications précédentes, **caractérisé en ce que** le polyoxy-alkylène A2) présente un indice d'OH dans une plage de 40 à 450 mg de KOH/g.

11. Mousse de polyuréthane pouvant être obtenue conformément à un procédé selon l'une quelconque des revendications précédentes.

12. Mélange à base de prépolymère polyuréthane, contenant les composants suivants :

A) un prépolymère polyuréthane, pouvant être obtenu à partir de

A1) diisocyanates de faible masse moléculaire, ayant une masse molaire de 140 à 278 g/mole, avec
A2) des polyoxyalkylènes ayant une fonctionnalité OH de deux ou plus,
A3) en option des composants supplémentaires réactifs avec des isocyanates, qui ne rentrent pas dans A2) ;

C) en option des oligomères hétérocycliques à 4 cycles ou 6 cycles de diisocyanates de faible masse moléculaire ayant une masse molaire de 140 à 278 g/mole ;
D) en option des catalyseurs ;
H) en option des polyisocyanates hydrophiles pouvant être obtenus par mise en réaction de

H1) diisocyanates de faible masse moléculaire ayant une masse molaire de 140 à 278 g/mole et/ou polyisocyanates pouvant être produits à partir de ceux-ci, ayant une fonctionnalité isocyanate de 2 à 6, avec
H2) des polyoxyalkylènes monofonctionnels ayant un indice OH de 10 à 250, et une teneur en oxyde d'éthylène de 50 à 100 % en moles, par rapport à la quantité totale des groupes oxyalkylène contenus,

le mélange à base de prépolymère polyuréthane, en particulier les composants A, C et H, ayant une teneur en isocyanates dans une plage de 2 à 8 % en poids et une teneur en groupes uréthane de 1,0 à 3,5 moles/kg, chaque fois par rapport à la quantité totale du mélange à base de prépolymère polyuréthane.

13. Utilisation du mélange à base de prépolymère polyuréthane selon la revendication 12 ou d'une mousse de polyuréthane selon la revendication 11, pour la fabrication d'un pansement, d'un article cosmétique ou d'un produit d'incontinence.

14. Mousse de polyuréthane selon la revendication 11, présentant au moins les propriétés suivantes :

a) un quotient de contrainte de rupture déterminée selon DIN EN ISO 527-2 et contrainte de rupture déterminée selon DIN EN ISO 527-2 à 20 % d'allongement (valeur F20) d'au moins 3,5 ;
b) une absorption de liquide ≥ 300 %, déterminée selon DIN EN 13726-1:2002 et indiquée en tant que pourcentage du poids sec ;
c) une densité, déterminée conformément à la méthode indiquée dans la description, entre 50 et 300 g/l ;
d) une contrainte de rupture, déterminée selon DIN EN ISO 527-2, d'au moins 50 kPa ;
e) une valeur F20, déterminée selon DIN EN ISO 527-2, d'au maximum 50 kPa.

15. Pansement, article cosmétique ou produit d'incontinence, pouvant être obtenu avec utilisation de mousses de polyuréthane selon l'une quelconque des revendications 11 et 14, ou produites selon l'une quelconque des revendications 1 à 10.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2143744 A
- EP 2470580 A
- EP 2585505 A
- EP 2632501 A
- US 3903232 A
- US 4137200 A
- US 5849850 A
- US 2006142529 A
- EP 3235520 A
- WO 2011006608 A
- EP 17156493

**In der Beschreibung aufgeführte Nicht-Patentliteratur**

- Ullmanns Encyclopädie der technischen Chemie. Verlag Chemie, vol. 19, 31-38